# EUROPEAN PATENT SPECIFICATION

(11) **EP 2 727 598 B1**
(45) Date of publication and mention of the grant of the patent: **13.06.2018**
(21) Application number: 12807925.8
(22) Date of filing: 29.06.2012
(51) Int. Cl.: A61K 33/04, A01G 7/00, A23L 3/358, A61K 8/23, A61P 5/16, A61P 9/14, A61P 11/00, A61P 17/00, A61P 17/02, A61P 29/00, A61P 31/10, A61P 37/08, A61Q 19/08, C02F 1/68, A61Q 19/00, C02F 1/28, C02F 1/70, C05D 9/02, A23K 20/20, A23K 50/80, A23K 50/75

(54) **BIOLOGICAL INORGANIC COMPOUND COMPLEX HAVING REDUCED OXYGEN AND HIGH REDUCING ABILITY**
BIOLOGISCHER ANORGANISCHER VERBINDUNGSKOMPLEX MIT REDUZIERTEM SAUERSTOFF UND HOHER REDUZIERUNGSWIRKUNG
COMPLEXE DE COMPOSÉ INORGANIQUE BIOLOGIQUE AYANT UNE TENEUR RÉDUITE EN OXYGÈNE ET UNE APTITUDE ÉLEVÉE À LA RÉDUCTION

(30) Priority: 01.07.2011 JP 2011147722
(43) Date of publication of application: 07.05.2014
(73) Proprietor: Nakayama, Eiki, Yokohama-shi, Kanagawa 226-0017 (JP); Hirakawa, Junko, Fukuoka-shi, Fukuoka 812-0038 (JP)
(72) Inventor: SUGAWARA Masahiro, Los Angeles, California 90095 (US)
(74) Representative: Schön, Christoph
(86) International application number: PCT/JP2012/066815
(87) International publication number: WO 2013/005698

(56) References cited:
- JP-A- 8 136 500
- JP-A- 60 168 311
- JP-A- 2000 197 872
- JP-A- 2003 327 537
- JP-A- 2006 304 671
- JP-A- 2007 020 519
- JP-A- 2010 155 808
- JP-A- 2010 158 179
- JP-A- 2010 259 351
- JP-A- 2011 157 280
- JP-U- 3 123 283
- OHYE H: 'Dual oxidase, hydrogen peroxide and thyroid diseases.' EXP BIOL MED vol. 5, no. 4, 23 April 2010, pages 424 - 433, XP008171793

## Description

### Technical Field

The present invention relates to a biological inorganic compound complex, specifically a biological inorganic compound complex having reduced oxygen and high reducing ability, which has a therapeutic effect on Graves' disease within a short period of time with few side effect, a pharmacological effect on other disorders, a cosmetic effect, a growth effect on animal and plant, and an environment-improving effect.

### BACKGROUND

The thyroid gland is an endocrine organ that produces the thyroid hormones. The thyroid hormones generally increase total body metabolism, and specifically, activate the metabolisms of digestive tract, heart, bones and nervous system. And hormonal secretion amount from the thyroid is regulated by a variety of hormones, especially regulated by thyroid-stimulating-hormone-releasing hormone (TRH) produced by the hypothalamus and regulated by thyroid-stimulating hormone (TSH) produced by the anterior pituitary according to a stimulation by TRH.

When the concentration of thyroid hormone is low, TRH and TSH are secreted to stimulate TSH receptors on the surface of the thyroid gland and to produce and secrete the thyroid hormones (T3 or T4). Further, the concentration of thyroid hormone increases, the secretions of TRH and TSH decrease and the production and secretion of the thyroid hormones decrease.

Now, the most common disorder on the thyroid gland functions is Graves' disease that develops because the autoantibody that binds to a receptor of thyroid-stimulating hormone (TSH) is generated and the generated autoantibody instead of TSH stimulates excessively the receptor, by which TSH is generated excessively and a variety of effects on the body occur.

The treatment of Graves' disease includes a medication, a radioactive iodine treatment and a surgical operation, but the medication is common today. In a medication, methimazole (MMI) and propylthiouracil (PTU) are used as an antithyroid agent, but it is known that these two drugs have side-effects; in which MMI causes an allergic reaction, an increase of a variety of enzyme levels in liver, a joint pain and an agranulocytosis in case of a severe condition (Non-patent document 1). Further, it is known that PTU causes severe liver damage (Non-patent document 2). The medication with either drug brings a very high burden to the body of which patients are suffering from Graves' disease. JP 2007/020519A (EIKI NAKAYAMA) 1 February 2007 and JP 2006/304671 A (EIKI NAKAYAMA) 9 November 2006 disclose plant mineral extracts for treating dermatological disorders or as food additive.

### Prior Art

### Patent Document

Non-patent dcoument 1 Cooper DS. (2005) Antithyroid drugs. N Engl J Med. 352:905-17.
Non-patent document 2 Cooper DS, Rivkees SA. (2009) Putting propylthiouracil in perspective. J Clin Endocrinol Metab. 94:1881-2.

### Summary of the invention

### Objects to be solved

Nevertheless, the above drugs are still used as the up-to-date medication of Graves' disease, in which a periodical measurement of thyroid hormone level and a regular and long-term administration of a proper dose of the drug are mandatory. This would bring physically and mentally a burden to the patient with additional risks due to side-effects. Accordingly, it is desirable if a drug could provide an effect with few side-effects and within a short period of time.

On the other hand, it is suspected that "active oxygen" affects a variety of diseases as one of causes. It is also getting clear that the active oxygen is generated in the body's metabolic processes and inhibits cell's functions and damages cells *per se,* and as results, an increase of active oxygen is involved in the mechanisms of many diseases, aging and so on. Oxygen is an inevitable element for living organisms but it oxidizes biological bodies and causes a variety of problems in another aspect. The problem caused by oxidation develops very gradually under normal physiological conductions, but if a balance between an oxidation reaction in the body and anti-oxidation action is out of control and the oxidation reaction progresses, it is understandable that a disease would develop. Such state is considered as an excess oxidation and the long-term and persistent oxidation stress causes a variety of diseases. Further, it also provides mal-effects on beauty, growths of animals and plants and environments.

The present invention is to solve the above problems, and according to a variety of experimental data obtained by Masahiro Sugawara at University of California, Los Angeles, a purpose of the present invention is to provide a biological inorganic compound complex having reduced oxygen and high reducing ability, which has a therapeutic effect on Graves' disease within a short period of time with few side-effects, and a pharmacological effect on other disorders, a cosmetic effect, a growth effect on animal and plant, and an environment-improving effect by suppressing effects (oxidation) due to an active oxygen that is considered as one of causes of other disorders.

### Means to solve the objects

The present invention provides a means that develops and produces a substance to achieve the above purposes. Materials comprising seaweeds including a kelp, a brown seaweed, a fucoi, a hijiki, a gagome-konbu, a sargassam sp.; and grasses and trees including a bamboo, a Japanese knotweed, wromwood, Houttuynia cordata, kudzu, a Stinking yew, a field horsetail, Sasa veitchii, a ceder, a cypress, a chestnut oak, a pine, are subject to ashing and then melted to provide a mixture. According to the process, most of four elements, oxygen (O; approximately 70%), carbon (C: approximately 18%), hydrogen (H: approximately 10.5%) and nitrogen (N: approximately 0.3) constructing more than 95% of a plant, are eliminated and almost all compounds may bring a toxic effect on a living organism disappears. Specifically, melting plants and making mixtures thereof, may produce a "biological inorganic compound complex" which has reduced oxygen and almost no toxic effect.

It is called now as a plant mixture substance (Bio Inorganic Elements (BIE)). The main elements of BIE are calcium, potassium, silicon and so on. Water is added to BIE and then it is boiled, filtered and extracted to provide water soluble plant mixtures (Reducing Inorganic Minerals; RIM). RIM, comprising sodium, potassium, chlorine, sulfur and so on as main elements, and a body fluid have a common mineral balance. BIE and BIM having a high reducing ability comprise plural sulfur compounds not binding to oxygen, which are not possible to be made by means of synthetic chemistry, and provide a biological inorganic compound complex containing almost all kinds of elements existing on earth.

According to the above composition, BIE comprises approximately -500 mV as an actual value of oxidation-reduction potential using TOA-RM-30P. Further, a liquid RIM comprises an actual value of oxidation-reduction potential between approximately -600 mV and -700 mV. Further, a RIM powder obtained by completely drying the liquid RIM comprises an actual value of oxidation-reduction potential between -80 mV and -300 mV.

A novel therapeutic pharmaceutical for hyperthyroid comprises a biological inorganic compound complex having reduced oxygen and high reducing ability. Further, the pharmaceutical can be applied to pharmacological treatments including atopic dermatitis, athlete's foot, burns, wounds, hemorrhoids, pneumonia, swelling, pain, itchiness and so on. Further, the biological inorganic compound complex having reduced oxygen and high reducing ability can be applied to a cosmetic or a cosmetic mixture. Further, the biological inorganic compound complex having reduced oxygen and high reducing ability can be applied to cultivations of plants and crops, feeds in animal husbandry/aqua-farming. Further, it can be applied to maintain food's freshness, extract umami, remove/clean contaminants, and sterilize. Further, a biological inorganic compound complex having reduced oxygen and high reducing ability can be applied to produce water having a common mineral balance of a body fluid.

### Effects of the invention

A biological inorganic compound complex of the present invention having reduced oxygen and high reducing ability comprises elements having a positive atomic valence and sulfur complex compounds can treat Graves's disease within a short period of time with few side-effects because of its reduced oxygen and high reducing ability, and can provide a pharmacological effect on other disorders, a cosmetic effect, a growth effect on animal and plant, and an environment-improving effect by suppressing an effect (oxidation) by an active oxygen that is considered as one of causes of other disorders.

### BRIEF DESCRIPTION OF THE DRAWINGS

Fig. 1 illustrates biosynthesis and secretion of thyroid hormones and a main signaling pathway in thyroid cells.
Fig. 2 illustrates DUOX molecule.
Fig. 3 illustrates a catalytic cycle of a peroxidase.
Fig. 4 illustrates a oxidation-reduction potential (ORP) meter. (UCLA) Fig. 5 illustrates main contents of RIM. (UCLA)
Fig. 6 is a table illustrating elemental analysis results of RIM powder by an X-ray microanalyzer (EDS) analysis. (Nittech Research Co. Ltd.)
Fig. 7 is a table illustrating elemental analysis results of BIE by an X-ray microanalyzer (EDS) analysis. (Nittech Research Co. Ltd.)
Fig. 8 illustrates a crystalline structure of powdery RIM; (a) is an electron microscopic photo of powdery RIM, and (b) is a figure of visualization after treating powdery RIM with 100% methanol and air-drying.
Fig. 9(a) is a 500 times enlarged figure of the crystalline structure of powdery RIM, and Fig. 9(b) is a 1000 times enlarged figure of the crystalline structure of powdery RIM. (Nittech Research Co. Ltd.)
Fig. 10 is a 2000 times enlarged figure of the crystalline structure of powdery RIM. (Nittech Research Co. Ltd.)
Fig. 11 (a) is a 500 times enlarged figure of the crystalline structure of powdery RIE, and Fig. 11 (b) is an 1000 times enlarged figure of the crystalline structure of powdery BIE. (Nittech Research Co. Ltd.)
Fig. 12 is a 2000 times enlarged figure of the crystalline structure of powdery BIE. (Nittech Research Co. Ltd.)
Fig 13 is a graph illustrating an X-ray diffraction result of RIM crystal. (UCLA)
Fig. 14 is a table illustrating qualitative results of RIM powder compound by an X-ray diffraction apparatus. (Nittech Research Co. Ltd.)
Fig. 15 is a table illustrating qualitative results of RIM powder compound by an X-ray diffraction apparatus. (Nittech Research Co. Ltd.)
Fig. 16 is a table illustrating qualitative results of BIE powder compound by an X-ray diffraction apparatus. (Nittech Research Co. Ltd.)
Fig. 17 is a table illustrating qualitative results of BIE powder compound by an X-ray diffraction apparatus. (Nittech Research Co. Ltd.)
Fig. 18 is a table illustrating qualitative results of BIE powder compound by an X-ray diffraction apparatus. (Nittech Research Co. Ltd.)
Fig. 19 is a graph illustrating identifications of main compounds in RIM powder by an X-ray diffraction apparatus. (Nittech Research Co. Ltd.)
Fig. 20 is a graph illustrating an identification of KNaS in RIM powder by an X-ray diffraction apparatus. (Nittech Research Co. Ltd.)
Fig. 21 is a graph illustrating water distribution and main ions in human body.
Fig. 22 is a graph illustrating identifications (Japan) of CaCO₃, SiO₂, KCl, NaCl and ((Ca, Na) (Si, Al))₄O₈ in BIE powder by an X-ray diffraction apparatus. (Nittech Research Co. Ltd.)
Fig. 23 is a graph illustrating an identification of KNaS in BIE powder by an X-ray diffraction apparatus. (Nittech Research Co. Ltd.)
Fig. 24 illustrates measurement results of an oxidation-reduction potential of non-crystalline RIM by a (ORP) meter.
Fig. 25(a) is a graph illustrating oxidation-reduction potential at each concentration of RIM, and Fig. 25(b) is a photo showing reducing property of RIM.
Fig. 26 is a graph illustrating an inhibition of guaiacol oxidation by RIM.
Fig. 27 is a graph illustrating an inhibition of iodination by RIM.
Fig. 28 illustrates a compound II level.
Fig. 29 is a graph illustrating a degradation enforced by RIM or MMI.
Fig. 30 illustrates examination results of thyroid functions.
Fig. 31 illustrates a cell localization diagnosis and a generation amount of H₂O₂ and oxygen in cultured cells.
Fig. 32 illustrates that RIM acts on a plasma membrane.
Fig. 33 illustrates a result in a study whether RIM acts on the most important signal of thyroid hormones formation from cell membrane-THS mediated cAMP generation.
Fig. 34 illustrates results of animal experiments.
Fig. 35 illustrates an animal experimental plan using rats.
Fig. 36(a) illustrates a structure of a mineral elution filter, and 36(b) is a cross section view illustrating a main body of an apparatus to produce mineral water.
Fig. 37 illustrates results of an experiment conducted by using an apparatus illustrated in Fig. 36.
Fig. 38 illustrates results of an experiment conducted by using an apparatus illustrated in Fig. 36.

### Examples

Next, the inventor of the present invention describes an example of the present invention.

### Introduction

A pharmaceutical comprising a biological inorganic compound complex having reduced oxygen and reducing ability is effective on a therapy of a variety of diseases including Graves' disease without side-effects. A compound complex of the present invention is produced by melting wild plants including seaweeds (grasses), wild grasses, trees and so on at higher temperature than 2000°C to make mixtures; and is a plant mixture active substance (bio-inorganic elements: BIE) Further, after addition of water to BIE and heating, and cooling down, it is filtered to provide a water soluble high reducing plant mixture (reducing inorganic minerals; RIM), which is either liquid or solid.

RIM has been used as a food preservative, skin care and prevention of cancer. This agent showed having the most potent reducing potential as far as observed so far. RIM Solution at 0.5% showed almost the same reducing potential as 100mM MMI in a testing using an oxidation-reduction potential (ORP) meter. RIM inhibited the reaction of peroxidase-H₂O₂ which was the first step of thyroid hormone formation analysis. In humans, an oral administration of RIM tocancer patients, non-thyroid cancer, showed a significantly higher incidence of hypothyroidism, 35%, than that of general population, 6.5%. No side-effect nor abnormal result in laboratory testings were noted.

The first study conducted is to characterize a RIM crystal by a powder X-ray diffraction means and with amorphous, non-crystal form, portions of RIM by ion exchange HPLC. The above two procedures are carried out at the Department of Chemistry, UCLA.

The second study conducted is to investigate the mechanism of antithyroid action of RIM *in vitro.* In cell-free system, the effect of RIM and MMI (a comparison drug) on the H₂O₂ generation system is examined by measurement of a Compound II level (an oxidizing product produced from the peroxidase/H₂O₂ reaction) and H₂O₂ degradation. For cellular experiments, human thyroid follicles in culture is used to examine the effects of RIM on H₂O₂ generation, iodide organification, and protein expression of DUOX and NOX4 (thyroid H₂O₂ generating enzymes).

In addition, FRTL-5 rat thyroid cells or normal human thyroid cell line of Nthy-ori-3-1 is used to measure intracellular cellular cAMP generated inresponse to RIM, because RIM affects plasma membrane structure and possible TSH receptor-mediated signals.

The third study conducted is animal experiments, using Sprague-Dawley rats, to obtain necessary information of RIM prior to use for human. Specifically, it is investigated whether RIM causes an irreversible hypothyroidism, thyroid atrophy or a goiter. In addition, a transdermal effect of RIM on rats is examined.

The final goal is to establish that the RIM is useful as a non-toxic and potent antithyroid agent for treatment of Graves' disease.

### Treatment of hyperthyroidism

The primary treatment method for Graves' disease is a thionamide drug therapy, a subtotal thyroidectomy, or a radioactive iodine therapy. In addition, a beta blocker, inorganic iodide or iodinate contrast media, lithium carbonate, potassium perchlorate glucocorticoids, and rituximab, a chimeric monoclonal antibody directed to human CD20, are secondary therapeutic options that are reviewed by Fumalora. However, these secondary treatment methods will never replace the primary treatment. Three primary methods described above have been worldwide and widely accepted and being in use among practicing physicians for more than a half century. In the United States, these are listed in the FDA drug therapy database and the most common treatments used for Graves' disease.

### Drug therapy with propylthiouracil (PTU) and methimazole (MMI)

Currently, MMI is the first choice of drug therapy because PTU has a lower adherence rate and a major toxicity that causes particularly a severe liver failure. The FDA recommends PTU as a second-line drug, to be used solely for patients who are intolerant of MMI, or for women in the first trimester of pregnancy. In general, MMI drug therapy has to be continued for 12 to 18 months to achieve its efficacy. MMI is known to have side effects, including allergic reactions, liver enzyme elevation, and arthralgia. Serious side-effects include agranulocytosis, aplatsic anemia and vasculitis.

Worldwide and well-known thyroid researches, but the field of antithyroid drugs, have significantly progressed. For better care of patients suffering from Graves'disease, a novel treatment method with fewer side-effects is urgently needed. The inventor of the present invention shall describe topics relevant to the proposed study.

### Steps of thyroid hormone formation

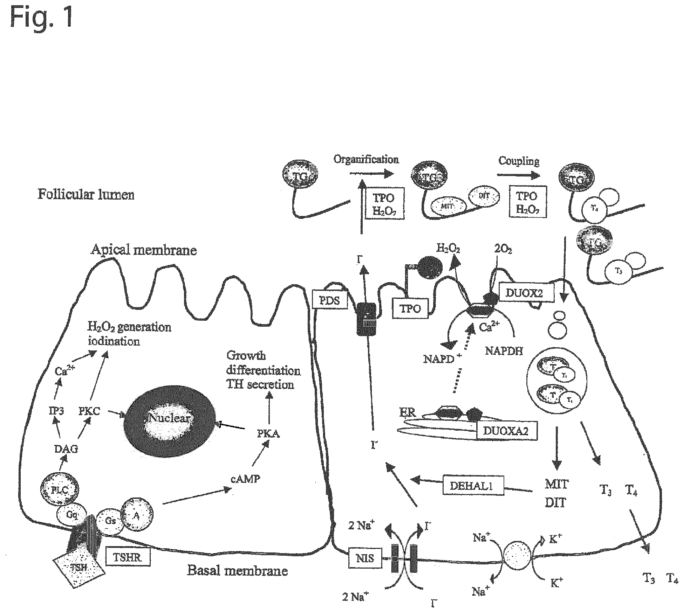

Fig. 1 illustrates a communication pathway of main signals in thyroid hormone biosynthesis, secretion and thyroid cells as shown below. Iodide is actively transported into thyrocytes by a sodium/iodide symporter (NIS) on the basolateral membrane and to the follicular lumen by, in part, pendrin (PDS/SLC26A4) at the apical membrane. Iodide is rapidly oxidized by TPO in the presence of H₂O₂ resulting in covalent binding to the tyrosyl residues of thyroglobulin (Tg) on the luminal side of the apical membrane. This step produces monoiodotyrosine (MIT) and diiodotyrosine (DIT). Then only properly spaced MIT and DIT in Tg participate in the coupling reactions to form thyroxine (T4) and tri-iodothyronine (T3). This reaction is also catalyzed by TPO with H₂O₂. The source of thyroid H₂O₂ is DUOX2 expressed in theapical plasma membrane coordinated with DUOXA2, DUOX maturation factor. Thyroid hormones are released into the circulation after digestion of Tg. MIT and DIT are deiodinated by an iodotyrosine dehalogenase 1 (DEHAL1). Thyroid hormone formation is predominantly regulated by thyrotropin (TSH). The binding of TSH to the TSH receptor activates both Gs and Gq proteins. The former activates the growth regulation, differentiation and thyroid hormone secretion, whereas the latter activates H₂O₂ generation and iodide binding to a protein through the phospholipase C-dependent inositol phosphate Ca₂+/diacylglycerol pathway.

The abbreviations in Fig. 1 stand for;
AC, adenyl cyclase;
cAMP, cyclic adenosine monophosphate;
DAG, diacylglycerol;
DEHAL1, dehalogenase 1;
DIT, diiodotyrosine;
DUOX, dual oxidase;
DUOXA, dual oxidase maturationfactor;
IP3, inositol trisphosphate;
MIT, monoiodotyrosine;
NIS, sodium-iodide symporter;
PDS, pendrin;
PKA, protein kinase A;
PKC, protein kinase C;
PLC, phospholipase C;
T3, tri-iodothyronine;
T4, thyroxine;
TG, thyroglobulin;
TPO, thyroid peroxidase;
TSH, thyrotropin;
TSHR, thyrotropin receptor

### DUOX hydrogen peroxide generating system in thyroid cells

The inventor of the present invention shall describe thyroid H₂O₂ generating system in detail, because the proposal involves RIM effects on H₂O₂ generation. To form thyroid hormone, H₂O₂ is required.

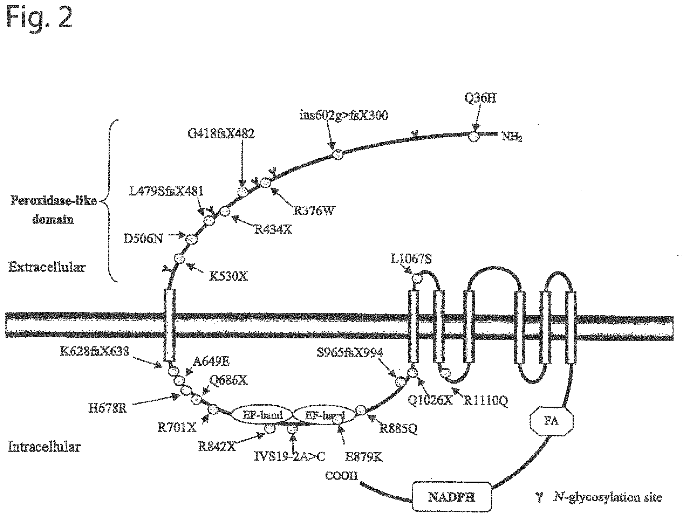

The detail of the H₂O₂ generating system in the thyroid gland is not described here, but the inventor of the present invention briefly describes the important aspects of thyroid H₂O₂ production. The existence of an NADPH oxidase in the thyroid gland has been known for a long time. It took 15 years to identify the dual oxidase (DUOX) protein as the major source of the thyroid H₂O₂. Originally, it was called as thyroid oxidase. There are DUOX1 and DUOX2 in the thyroid gland. DUOX2 but not DUOX1 is responsible for thyroid H₂O₂ production. For transportation of DUOX enzymes to the apical membrane, their maturation factors, DUOX1A and UOXA2, are required. DUOX is one of the six NADPH oxidase (NOX) enzymes, and its isoform shares 50% homology with NOX2. DUOX molecule is shown in Fig. 2.

### Structural models of DUOX2 protein and sites of reported mutations

Mutation sites at the amino acid level are indicated by small closed circles. Arrows illustrates changes in amino acids resulting from the mutations. The peroxidase-like domain is located in the N-terminal extracellular portion. Two EF-hands for Ca₂+-binding sites are on the first long intracellular loop. FAD and NADPH binding cavities are on the intracellular C-terminal portion. FAD, flavin adenine dinucleotide;

### NADPH, nicotinamide adenine dinucleotide phosphate

The DUOX protein has 7 transmembrane domains, and the seventh domain has peroxidase-like structure. It is controversial whether DUOX functions as peroxidase, but characteristically, DUOX has an EF-hand for calcium binding that is the major stimulant of the enzyme activity. As seen in all NOX members, it contains hemein the transmembrane domains. A function of DUOX is mono-electron transportationfrom NADPH to O₂ via FAD and heme. NADPH cannot be replaced with NADH in the DUOX system. When an electron is given to oxygen, it forms super oxide anion (O₂⁻). However, a rapid conversion from O₂⁻ to H₂O₂ by superoxide dismutase escapes biological detection of O₂⁻. It is interesting to examine whether addition of RIMdisturbs the electron transport system.

### Controlling factors of DUOX

Calcium is the most potent stimulator of DUOX through the binding to an EF-hand of the DUOX molecule as described above. In addition, protein kinase C activates DUOX2 but not DUOX1. TSH stimulates almost all steps of thyroid hormone formation. However, it does not stimulate DUOX2 in thyroid cells except rat thyroid cell line of PCCI3. Thyroid DUOX1 that is stimulated by TSH does not significantly contribute to thyroid H₂O₂ generation. There are no potent and specific inhibitors of NOX enzymes. Most inhibitors are nonspecific. An iodonium-derivative, diphenylene iodonium (DPI), has been commonly used, because this compound abstracts an electron from an electron transporter and forms a radical. And this compound inhibits the respective electron transporters. The antioxidant, N-acetylcysteine, that is commonly used inhibits DUOX protein expression in mouse thyroid.

### Methods of measuring DUOX activities

There are two methods of measuring DUOX activity. Mühlbauer describes a simplified method to detect DUOX activity from thyroid particulate fraction (10,000 xg pellet). Incubation medium contains calcium, FAD and NADPH. The amount of H₂O₂ production was measured at the end point of activity measurement. Rigutto et al. measured H₂O₂ production, using cultured Cos-7 cells co-expressing DUOX/DUOXA, in the presence of low concentration of 0.8nM PMA for DUOX2 activity. To measure DUOX1 activity, FSK was added to the incubation medium. This method is used for measurement of DUOX2 activity and H₂O₂ production in human thyroid cells without transfection, the process of introducing nucleic acids of such as gene and virus into cells for growth, with DUOXA2 because DUOXA2 (maturation factor) is present in human thyroid cells.

### Mutation of DUOX genes causing hypothyroidism in human

Abnormal DUOX causing hypothyroidism is known. Since Moreno et al. reported the first four cases of DUOX2 mutation in 2002, 22 cases of patients with DUOX2 mutation have been reported so far. According to Moreno et al., biallelic mutations lead to permanent hypothyroidism, whereas monoallelic mutations result in transient hypothyroidism. However, Maruo et al. reported a transient hypothyroid case despite biallelic mutations causing truncated DUOX2 protein. This report suggests that the existence of an alternative mechanism to produce H₂O₂ when DUOX2 loses its function.

### H₂O₂ measurement

Measurement of H₂O₂ is crucial in this project. It is essential to use valid methods. The inventor of the present invention describes here methods of H₂O₂ detection to be used in the experiments. Measurement of H₂O₂ depends on the source of samples, such as a biological fluid, which is cell free medium and in vitro reaction mixture, or an intracellular compartment. A measurement of H₂O₂ from biological fluid is relatively easy. However, it is difficult to measure intracellular H₂O₂, since H₂O₂ probes have to cross cell membrane first. Also, most commonly used probes cross-react with other ROS or free radicals. However, a progress has been made to overcome some of the problems.

### H₂O₂ assays from biological fluids

### i) Amplex Red [N-actyl-3,7-dihydroxyphenoxazine] method using Amplex red reagent that is a fluophor having extremely high sensitivity.

The basic principle is that H₂O₂ reacts with Amplex Red (Molecular Probes - Invitrogen) at a stoichiometry of 1:1 in a reaction catalyzed by horseradish peroxidase (HRP). This generates the highly fluorescent product resorufin that exhibits a maximum of fluorescence emission at a wave length of 587nm and maximum excitation at 563nm. Its extinction coefficient is 54,000M⁻¹ cm⁻¹. This assay is highly sensitive and selective experiment for H₂O₂ and can detect a lesser amount than 50nM H₂O₂. This method is commonly used for ng ability comprising a complex compound consisting of elementspic microplate format.

### ii) TMB (3,5,produces 3,5"-tetramethylbenzidine) method

The TMB is oxidized by horseradish peroxidase and H₂O₂ to produce TMB cation free radicals that can be measured at 653nm with ε = 3.9x10⁴ M⁻¹ cm⁻¹. Further oxidation with HRP produces diimine (absorbance at 450nm with ε = 5.9x10⁴ M⁻¹ cm⁻¹). This is a simple method with high sensitivity.

### Intracellular detection of H₂O₂

iii) 5,6-Chloromethyl-2',7'-dichlorodihydrofluorescein diacetate (CM-DCFH-DA) method CM-DCFH passively diffuses into cells, and intracellular processing of this compound by ROS generates highly fluorescent CM-DCF that can be measured atemission of 530nm and excitation of 488nm. This is easy to perform and sensitive. Therefore, it is used widely to detect intracellular H₂O₂ that is the major intracellular ROS. The probe, however, is not specific to H₂O₂, since it reacts with other oxidants.

### iv) HyPer fluorescent method as the most specific intracellular H₂O₂ detection method

HyPer, a genetically encoded ratiometric sensor, is highly selective for H₂O₂ over other than ROS. HyPer consists of the bacterial H₂O₂-sensitive transcription factor OxyR fused to a circularly permuted YFP. Cysteine oxidation of the OxyR part induces aconformational change that increases emission excited at 500nm (YFP500) and decreases emission excited at 420nm (YFP420). This change is rapidly reversible withinthe reducing cytoplasmic environment, allowing dynamic monitoring of intracellular H₂O₂ concentration. This method is suitable to trace H₂O₂ production by a fluorescent microscope. HyPer vector is now commercially available (Wako Chemicals Inc., Richmond VA , distributer of Evrogen)

### H₂O₂ reaction with peroxidase: spectral aspect

The reaction between H₂O₂ and thyroid peroxidase (TPO) is the first step of thyroid hormone formation. The following four mammalian peroxidases catalyzeoxidation of iodide: myeloperoxidase, eosinophil peroxisase, lactoperoxidase (LPO) and thyroid peroxidase TPO.

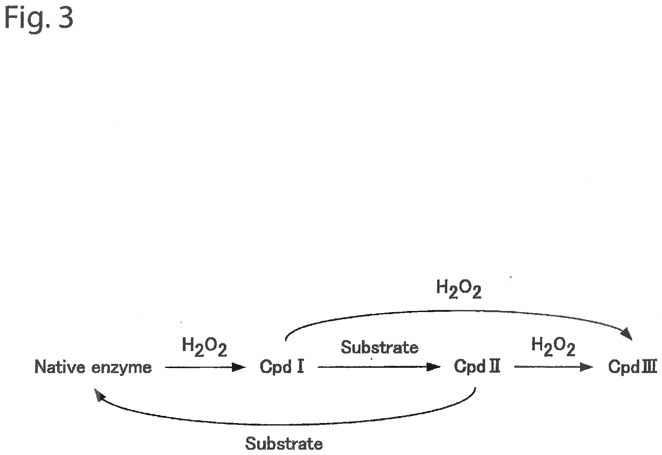

These four peroxidases have a similar structure each other based on open reading frame analysis. The inventor of the present invention now focuses on LPO and TPO, since they catalyze thyroid hormone formation (because of catalytic effect). As other peroxidases have, LPO and TPO have heme in the molecule, a critical element of electron transfer (redox) of peroxidase enzymes. The proximal heme ligands in LPO are His468 and Asn554, and in TPO His494 and Asn579. The inventor of the present invention describes herespectral properties of LPO- H₂O₂ reaction because purified TPO is difficult to obtain. However, similar spectral responses can be expected from TPO. The catalytic cycle of peroxidase is shown in Figure 3.

### Formation of compound I, compound II and compound III by LOP and H₂O₂

The optical spectrum for native LPO shows a Soret band at 412nm (ε = 114 000 M⁻¹•cm⁻¹).
Exposure to equimolar or two fold excess of H₂O₂ produces the porphyrin-radical Cpd I that is characterized by absorptions at 420 or 416nm.

In the presence of peroxide only, Cpd I converts spontaneously (within 200 msec) into a relatively stable species Cpd II that can be detected at 430nm. Exposure to slight peroxide excess rapidly inactivates LPO and generates Cpd III (with bands at 424nm).

The Cpd I is responsible to catalyze iodination of tyrosine residues of thyroglobulin and coupling of iodityrosines, the last step of thyroid hormone formation. In addition, the isomer Cpd I-[FeIV=O; aa⁺?] or Cpd II, whose oxidizing power is lower than that of Cpd I, are possibly catalyze iodotyrosine coupling.

### Oxidation-reduction potential (ORP) meter

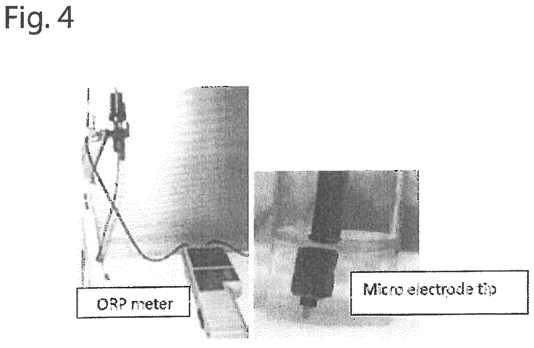

Masahiro Sugawara, UCLA, extensively use ORP meter in the proposed study. Therefore, the inventor of the present invention describes the sensitive electrode for the ORP meter. The inventor of the present invention uses calibrated pH and redox microelectrodes (ORP-146C microelectrode; Lazar Laboratories Inc., Los Angeles, CA) connected to a portable pH-redox meter (model 6230, Jenco Inc., San Diego, CA). The basic principal of this instrument is to measure electron movement to or from platinum microelectrode in solution. For example, a reducing agent is an electron donor. Thus, the electrode receives negatively charged electrons, which makes minus reading of mV. In contrast, an oxidizing agent (an electron acceptor) gives positive mV. Fig. 4 is the picture of this instrument. The advantages of this electrode are that it is very sensitive and it can be applicable to 10 to 20µL sample size.

However, an ORP-meter, RM30P (DKK-TOA Corporation) is used in Japan to obtain the actual measurement values that are data shown in the present invention.

### Significance

Graves' disease is one of the common thyroid diseases in VA patients. A number of patients with Graves' disease are suffering from drug side effects and forced to take radioactive iodine therapy. The study proposed here involves clinically very important but completely forgotten or ignored thyroid research area-antithyroid agent. Once physicians or patients experience serious drug side effects, the necessity of developing a new agent can easily be understood. The outcome of this research should significantly improve treatment of Graves' disease not only for VA patients but also for non-VA patients. The agent of reducing inorganic mineral (RIM) consists of minerals without organic chemicals and an oxygen-poor and potent reducing agent. Thus, fewer side-effects can be expected. RIM therapy likely replaces a 60-year-old-drug therapy of Graves' disease in the future.

### Example

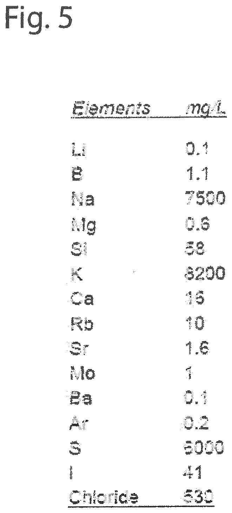

Fig. 5 illustrates a result of an elemental analysis of 3% RIM solution conducted by Masahiro Sugawara (UCLA). According to Fig. 5, sodium, potassium and sulfur are predominant followed by chlorine. An analysis of 15% of RIM solution that is conducted on demand at Japan Food Analysis Center shows; sodium 3.35%, potassium 2.41% (by atom absorption) chlorine 3.4% (by potentiometric titration), and sulfur 1.79% (by barium weight method), and the trend is almost the same.

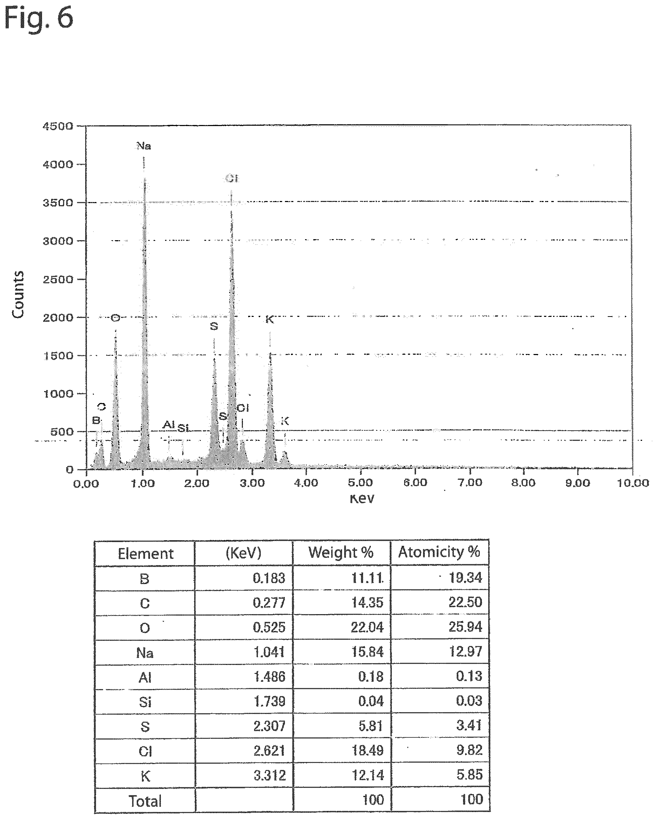

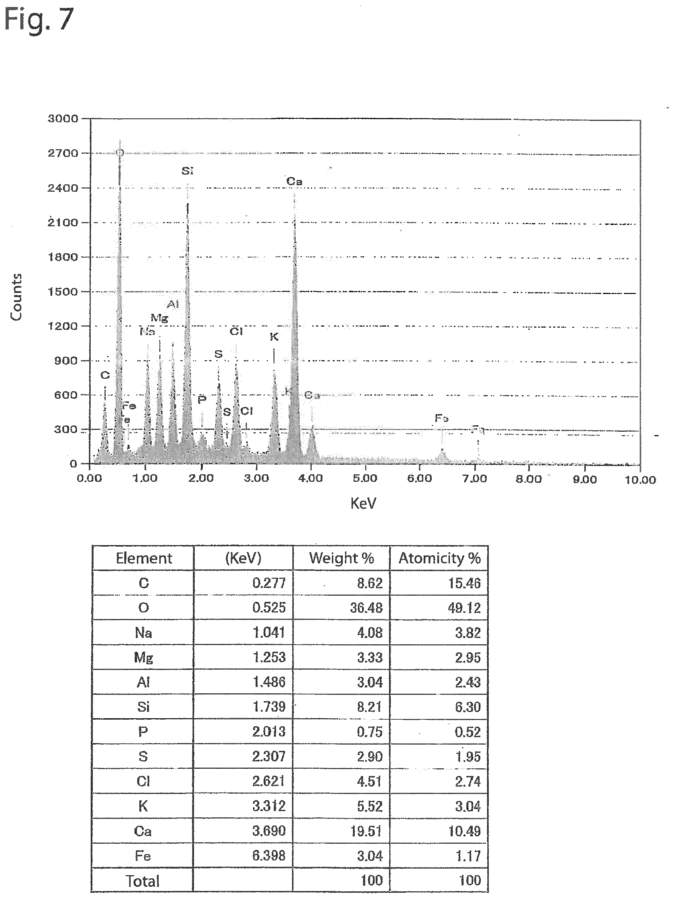

Next, analytical results of RIM powder and BIE powder using an X-ray micro-analyzer, conducted by Nittech Research Co. Ltd. are shown in Fig. 6 and Fig. 7.

As shown in Figs., RIM contains 15.8% sodium, 12.1% potassium, and 18.4% chlorine, and the result is the same result as by Japan Analytical Center but sulfur 5.8% is less and controversially, it contains 11.1% boron, 14.3% carbon and 22.0% oxygen.

Further, BIE powder contains 18.5% potassium, 8.2% silicon, 5.5 % potassium, 4.5% chlorine, 4.0% sodium, and 2.9% sulfur. In addition, it contains 36.4% oxygen and 8.6% carbon.

Accordingly, even heated up to a mixture state, oxygen and carbon cannot be completely eliminated. However, oxygen content of a plant is fundamentally approximately 70% and carbon thereof is approximately 18%, and it is observed that hydrogen and nitrogen are almost are eliminated and oxygen and carbon are reduced approximately by half, and it is considered rather in reduced oxygen condition, specifically it changed from oxygen rich to oxygen poor inorganic compound complex.

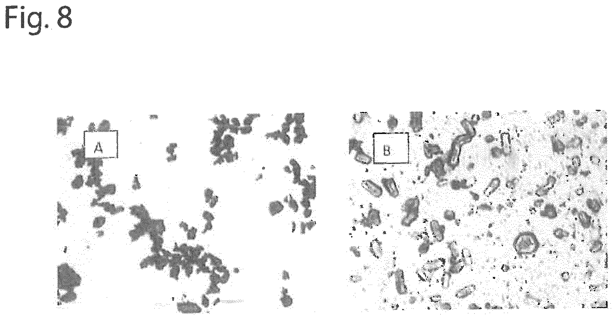

Fig. 8 illustrates a crystalline structure of powdery RIM obtained at UCLA.

Fig. 8(A) is an electro microscopic photo of powdery RIM and Fig. 8(B) is taken after treating powdery RIM with 100% methanol and then drying and visualization so that the crystalline structure can be clearly confirmed.

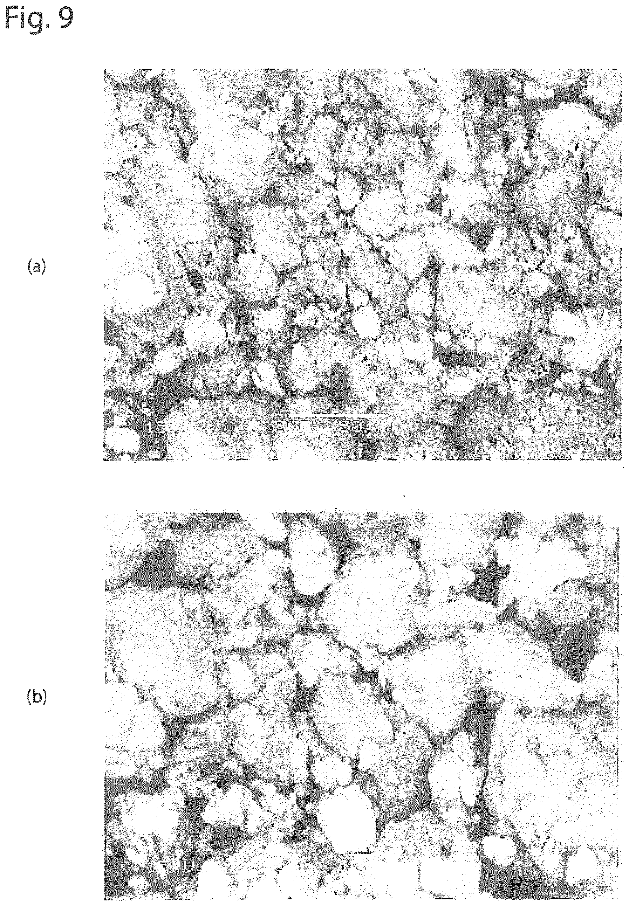

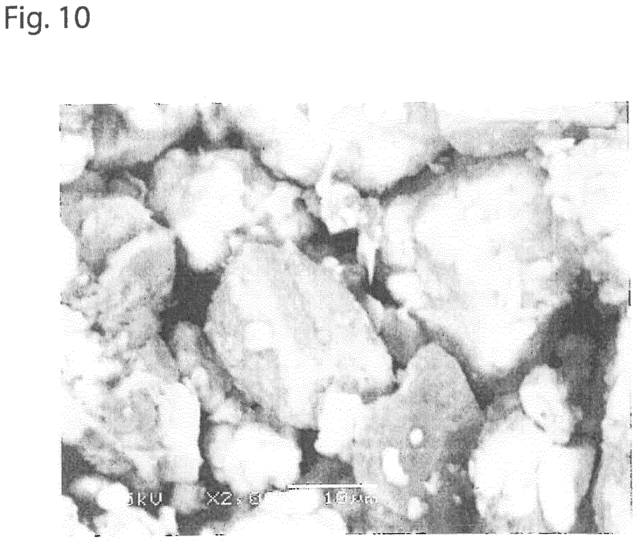

Fig. 9 and Fig. 10 illustrate a crystalline structure of RIM and RIE obtained by a scanning electron microscope at Nittech Research Co., Ltd.. Fig. 9(a) is a 500 magnification composition image, and 9(b) is an 1000 magnification composition image. Fig. 10 is a 2000 magnification composition image,

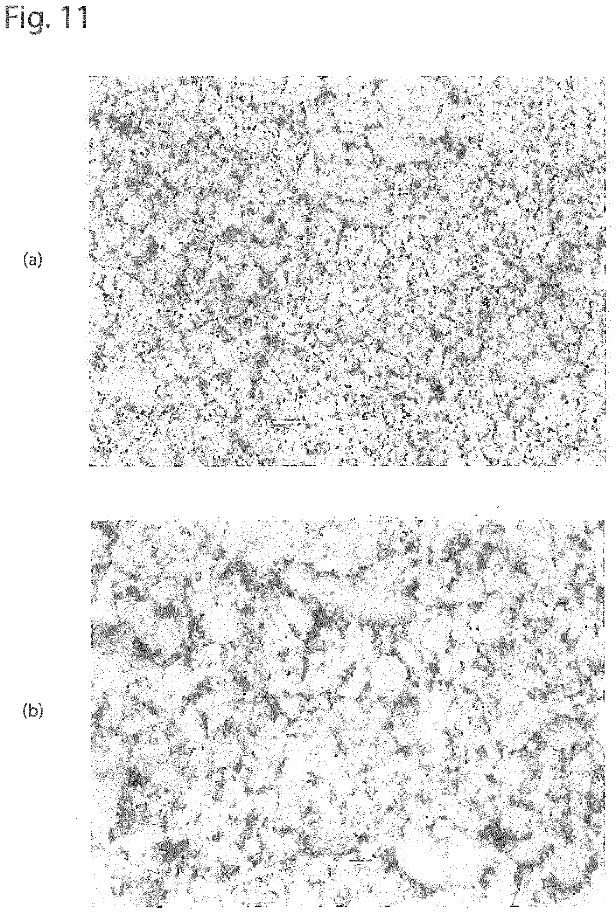

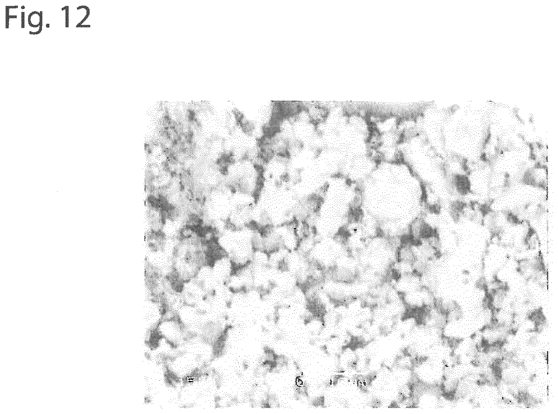

Fig. 11 and Fig. 12 illustrate composition images of a plant mixture material taken by a scanning electron microscope (SEM) at Nittech Research Co., Ltd.. Fig. 11(a) is a 500 magnification composition image, and 11(b) is an 1000 magnification composition image, and Fig. 12 is a 2000 magnification composition image,

### Crystal analysis of RIM crystal

Next, RIM crystal is analyzed by using X-ray diffraction.

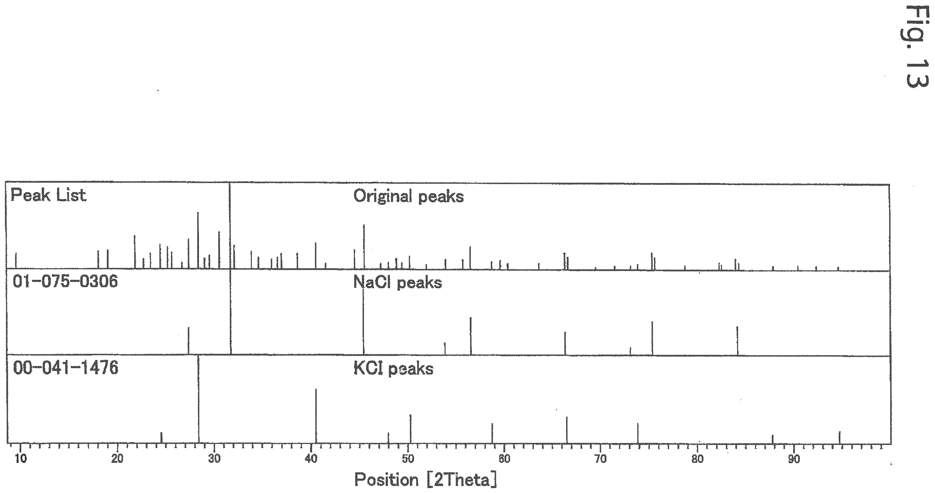

Fig. 13 is a graph showing an X-ray diffraction result of RIM crystal in UCLA.

The graph on the top in Fig. 13 represents original peaks; middle and lower graphs are major peaks abstracted from the original peaks. The powder analyzed by this method shows multiple peaks, and two major identified peaks are NaCl and KCI. Some of the peaks from the remaining peaks could probably be from sodium dithionate (Na₂S₂O₄), aphitalite (NaK₃SO₄), or sodium sulfate (Na₂SO₄).

However, most of the remaining multiple peaks could not be identified from JCPDS-ICCD database.

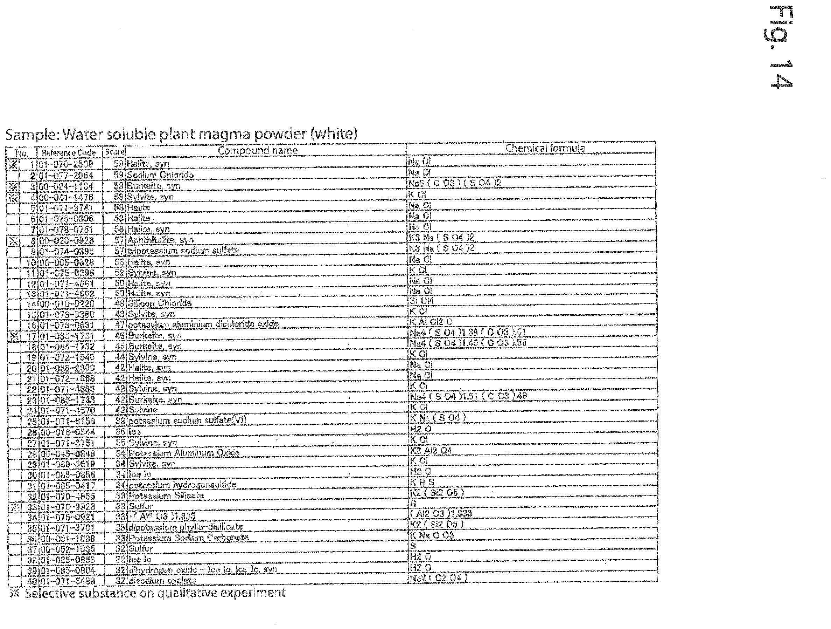

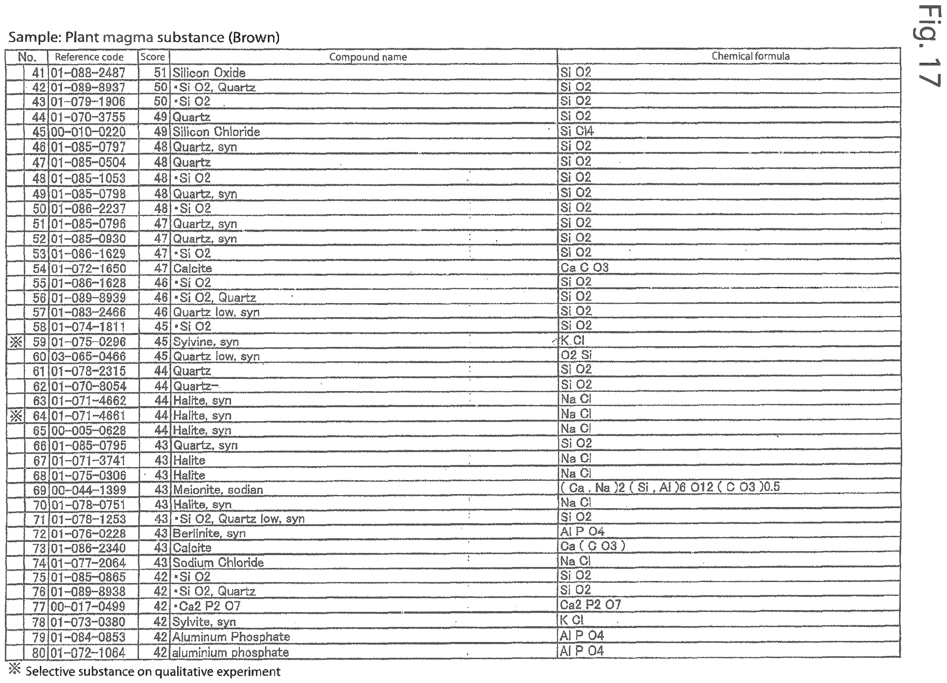

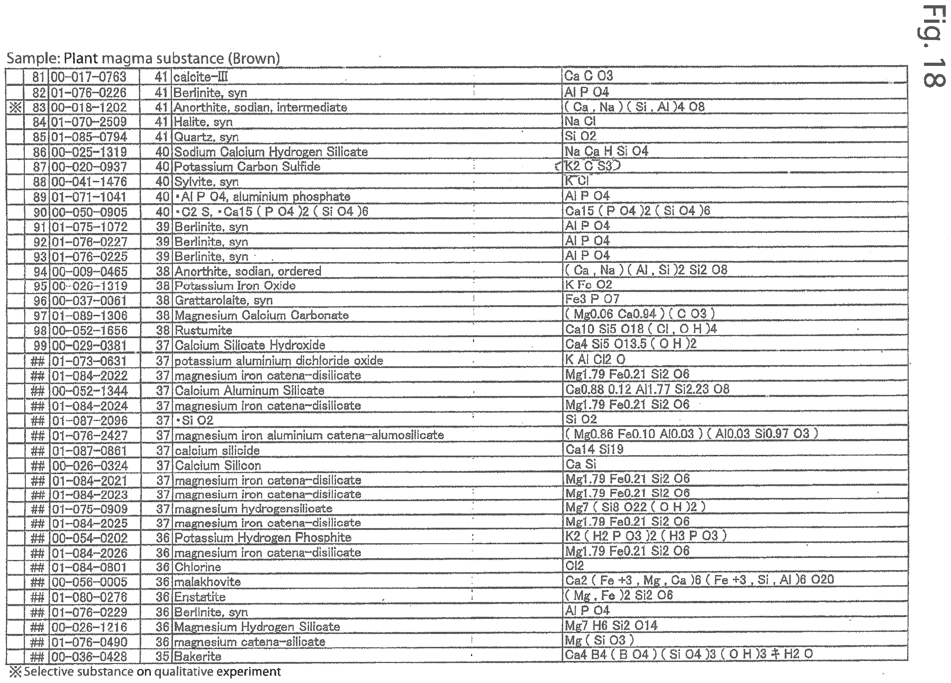

Next, referring Fig. 14 - Fig. 20, Fig. 22 and Fig. 23, quantitative results and identifications of RIM powder and BIE powder compounds are illustrated.

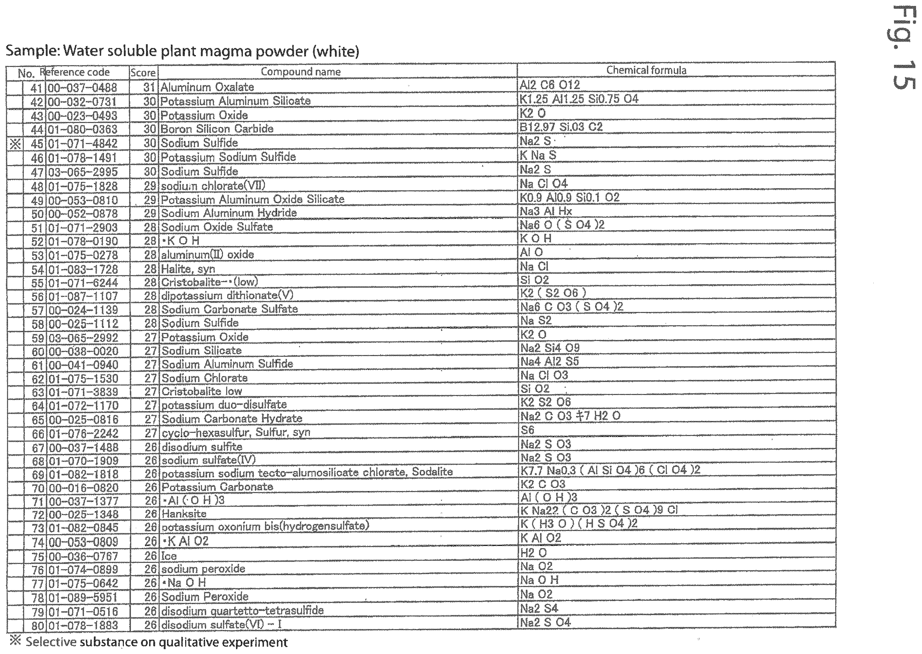

Fig. 14 and Fig. 15 are tables illustrating qualitative results of RIM powder compound by an X-ray diffraction apparatus.

As shown in Fig., RIM powder contains peaks assigned to K₃Na(SO₄)₂, Na₄(SO₄)_{1.39}(CO₃)_{0.61}, Na₄(SO₄)_{1.45}(CO₃)_{0.55}, Na₄(SO₄)_{1.51}, (CO₃)_{0.49}, KNa(SO₄), KHS, S, Na₂S, KNaS, SiO₂, NaS₂, S₆, Al(OH)₃ and Na₂S₄.

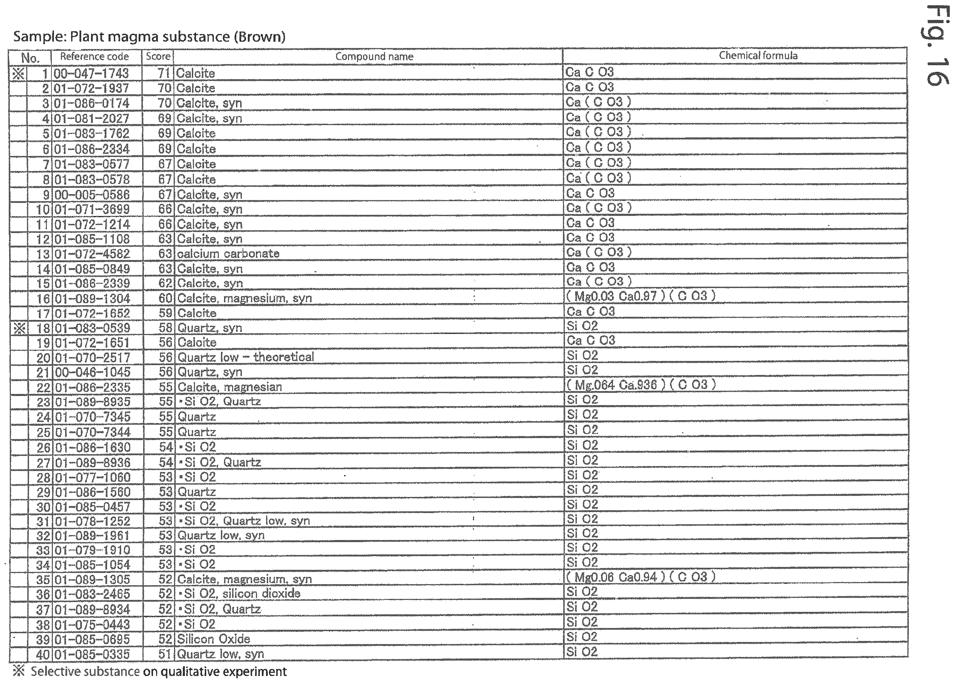

Fig. 16 - Fig. 18 are tables illustrating qualitative results of BIE powder compound by an X-ray diffraction apparatus.

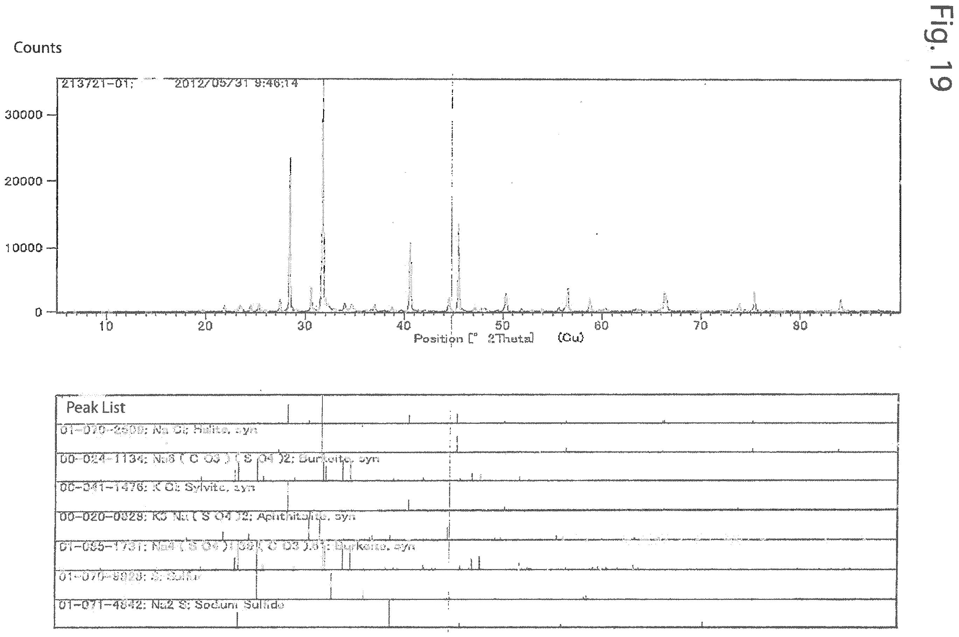

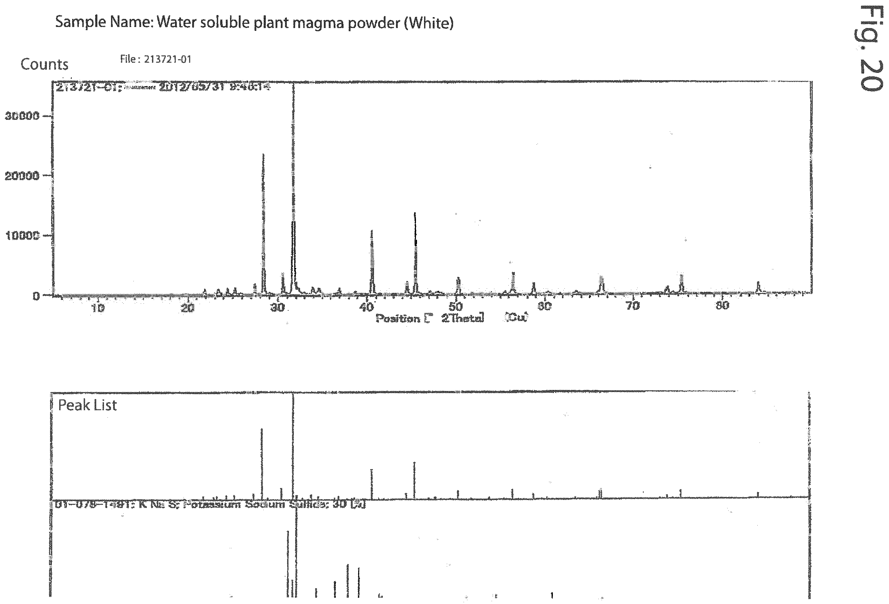

As shown in Fig., it is clear that BIE powder contains K₂CS₃. Further, Fig. 19 is a graph illustrating identification of main compounds in RIM powder by an X-ray diffraction apparatus, and Fig. 20 is a graph illustrating identification of KNaS in RIM powder by an X-ray diffraction apparatus,

As shown Figs., BIE powder contains peaks assigned to such as NaCl, Na₆(CO₃)(SO₄)₂, KCl, K₃Na(SO₄)₂, Na₄(SO₄)₁.₃₉(CO₃)₆, S and Na₂S. In addition, as shown in Fig. 20, it is clear that BIE powder contains a peak assigned to KNaS.

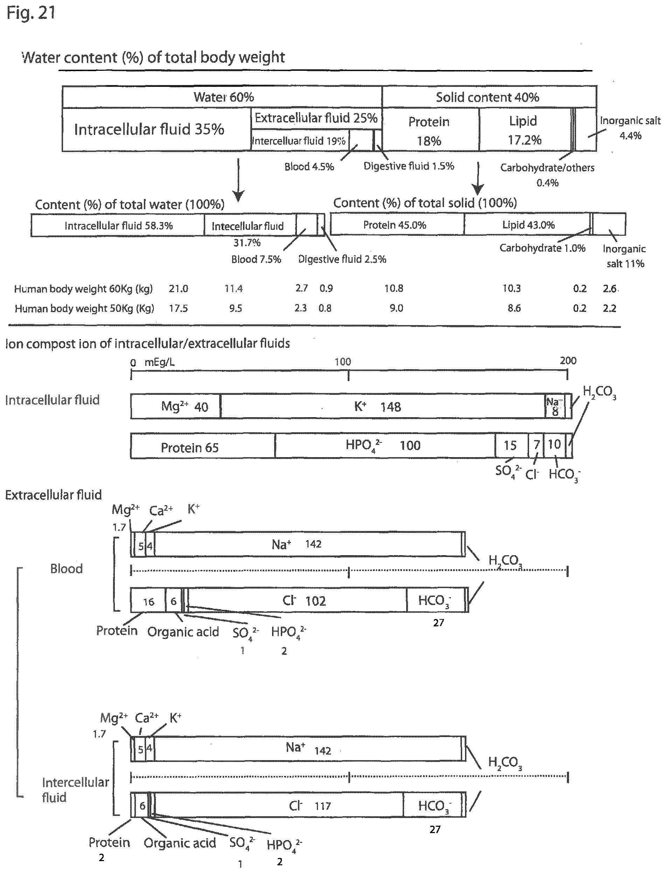

Fig. 21 is a graph illustrating water distribution and main ions in human body.

As shown in Fig., it is clear that there are common aspects between an ion composition of intracellular fluid and an extracellular fluid and mineral contents of RIM or BIE.

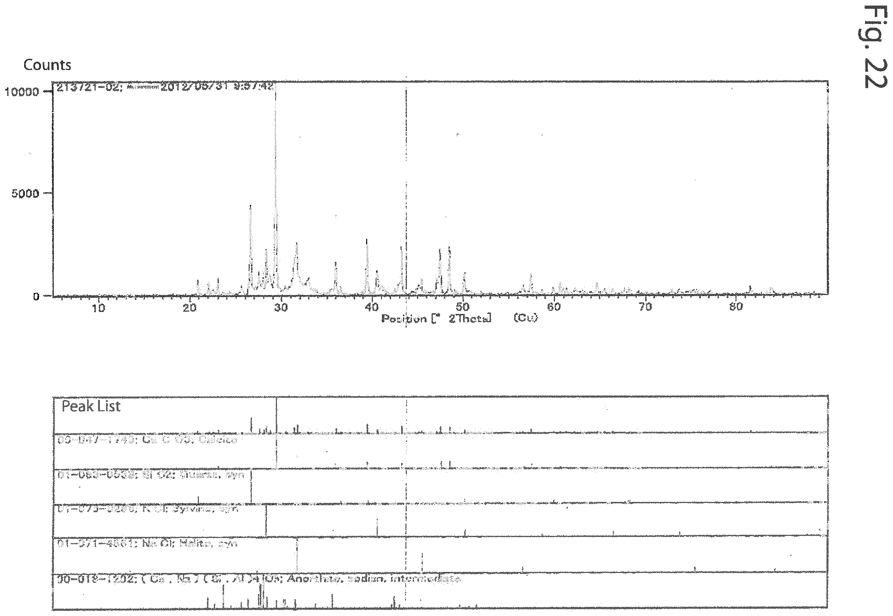

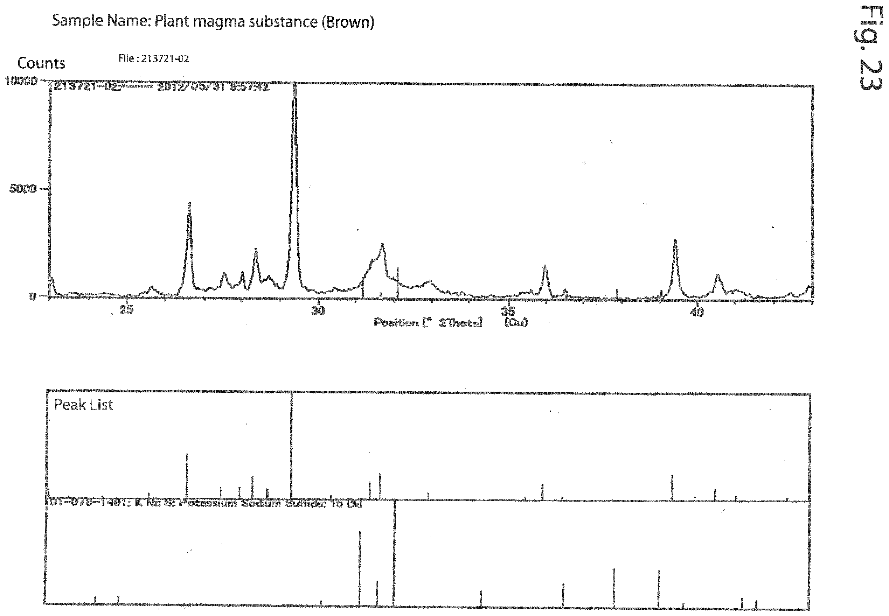

Next, Fig. 22 illustrates identifications of CaCO₃, SiO₂, KCl, NaCl, (Ca, Na)(Si, Al)₄O₈ in BIE powder by an X-ray diffraction meter, and Fig. 23 illustrates an identification of KNaS in BIE powder by an X-ray diffraction meter,

As shown Fig., BIE powder contains peaks assigned to such as CaCO₃, SiO₂, KCL, NaCl, (Ca, Na)(Si, Al)₄O₈. Fig. 23 is a graph illustrating an identification of KNaS in BIE powder by an X-ray diffraction apparatus.

As shown in Fig., it is clear that BIE powder contains a peak assigned to KNaS.

### Analysis of amorphous RIM

Next, amorphous components of RIM were analyzed.

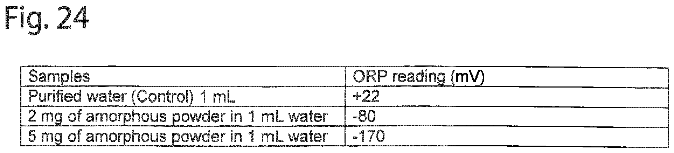

The inventor of the present invention measured oxidation-reducing potentials of amorphous components by the ORP meter. The results of ORP meter analysis are shown in Fig. 24.

Referring to Fig. 24, ORP of purified water (control) 1 ml was +22mV, but ORP of 2mg amorphous powder in 1 mL water solution showed -80mV and ORP of 5mg amorphous powder in 1 mL water showed -170mV.

These results showed that amorphous materials of RIM might contain the core of reducing activity.

### Reducing potential of RIM

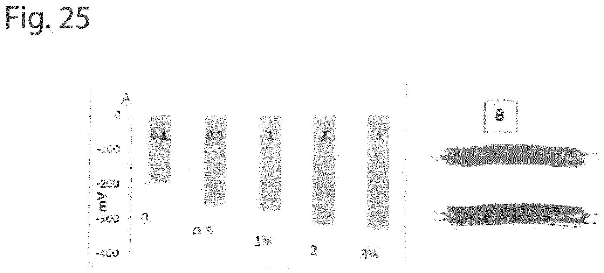

Fig. 25(a) is a graph showing oxidation-reducing potential of RIM at each concentration and Fig 25(b) is a photo showing reducing activity of RIM.

A graph in Fig. 25(a) shows oxidation-reducing potential of RIM at each concentration As shown in Fig., RIM at 2-3% concentration shows not less than -300mV reducing potential, which is higher than reducing potential of metymazol (MMI) that is used on treatment of hyperthyroidism.

In addition, as shown in Fig. 25(b), when a rusted metal spring (top photo) was exposed 2% RIM for a week, the rust of the metal spring was removed (bottom photo), by which it is obvious that RIM has potent reducing-potential.

Further, boiling or freezing RIM did not decrease its reducing potential and a stable condition has been maintained over three months without losing reducing-potential.

### Inhibition of peroxidase-H₂O₂ reaction by RIM

### (i) Guaiacol oxidation method

This assay is used to measure thyroid peroxidase (TPO) and LPO activities.

Guaiacol oxidation by TPO (LPO) and H₂O₂ produces tetraguaiacol on which OD (absorbance) at 470nm is measured.

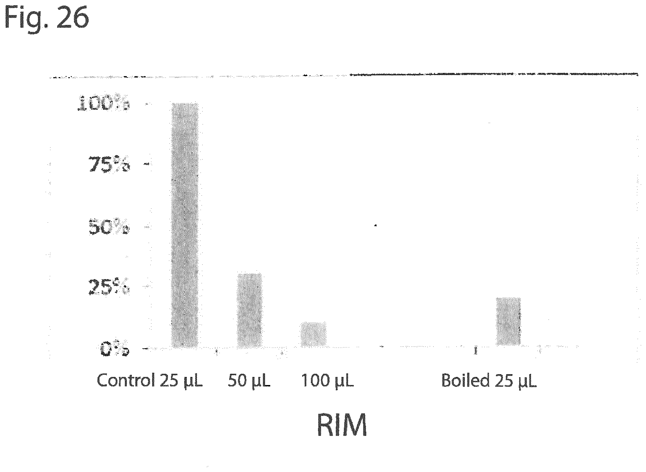

As shown in Fig. 26 that is a graph showing inhibition of guaiacol oxidation by RIM, it is obvious that RIM inhibits LPO-catalyzed guaiacoloxidation in a dose response manner.

It is shown that even boiled RIM inhibits guaiacol oxidation.

The reaction mixture contains 300µM guaiacol, 10µg LPO (Worthington Biochemical, Lakewood, NJ), 1% RIM (0-100µL) and 1 mM H₂O₂ in a total 1 mL of 0.05M phosphate buffer, pH 7.0. The absorbance at 470nm measured at 1 min is considered as a peroxidase activity. The results are means of three samples. SD values were eliminated from this graph because they were too small.

### (ii) Iodination assay method

Thyroid hormone formation involves iodination of thyroglobulin by peroxidase and H₂O₂. Then, radioiodination of bovine serum albumin (BSA) is carried out in vitro in the presence of LPO and H₂O₂.

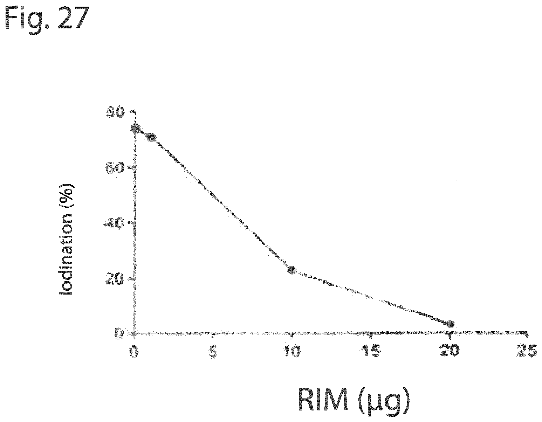

The reaction mixture contained 5mg BSA, a trace amount of 125I-iodide (20,000cpm), 100nM KI, 1mM H₂O₂ and 20µg LPO is incubated at 37° C for 10 min, and then 100µL of cold 1 M trichloroacetic acid (TCA) is added to stop the reaction. The pellet of TCA precipitate is washed with PBS, and the radioactivity of the pellet is counted. The results are shown in Fig. 27.

Fig. 27 shows an inhibition of iodination by RIM.

As shown in Fig., RIM inhibits LPO-mediated iodination within narrow ranges. Thus, antithyroid action of RIM is confirmed under a physiological condition.

### Inhibition of peroxidase reaction by RIM

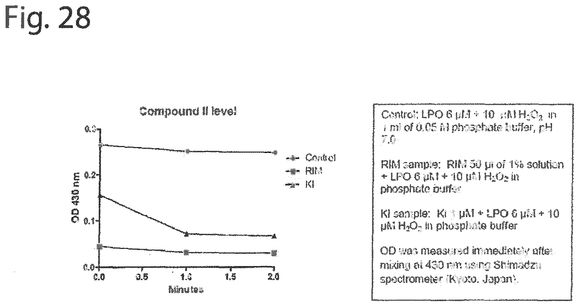

The inventor of the present invention examined the effect of RIM on Compound II formation, an oxidizing product from the peroxidase- H₂O₂ reaction, and degradation of H₂O₂ in vitro to analyze the inhibition of peroxidase reaction by RIM. Compound I is the most active oxidation free radical and responsible for iodide oxidation, but it is difficult to measure due to a very short life of 0.2 sec. Compound II, however, is relatively stable and can be measured at OD of 430nm. The compound II level is well maintained after mixing LPO and H₂O₂ (Referring to Fig. 28). When 50µL of 1% RIM is added to the LPO- H₂O₂mixture, compound II formation is completely inhibited, and an inhibition of compound I or compound II formation or both is suggested. Since the compound II is derived from the compound I, the inhibition of compound I is also understandable. KI at 1µM partially inhibits compound II formation because compound I and likely compound II are utilized for iodide oxidation.

### Degradation of H₂O₂ by RIM

An inhibition of LPO- H₂O₂ reaction by RIM can be explained by measuring a degradation of H₂O₂ by RIM in a cell-free system.

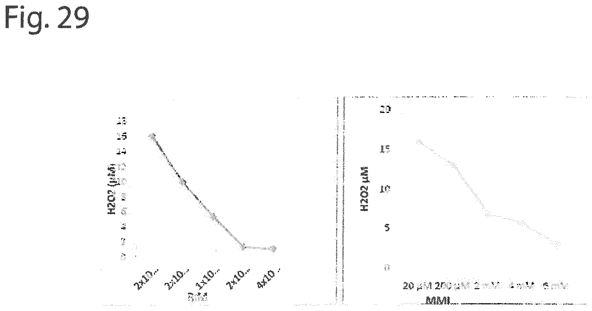

In measurement, 20µM H₂O₂ is mixed with RIM or MMI and then a H₂O₂ level is measured after incubation. A result is shown in Fig. 29.

Fig. 29 shows an enhanced degradation by RIM or MMI.

In this measurement method, RIM is mixed with 20µM H₂O₂ in a total volume of 50µL of reaction buffer in a 96-well plate, and the mixture is incubated for 30 min at 37°C. Then, Amplex red (Invitrogen) is added and incubated for 30 min in a dark room. The absorbance at 560nm is measured by a microplate reader (Molecular Devices, Sunnyvale, CA). As shown in Fig., a standard curve is constructed from 0 to 20µM H₂O₂, and wherein 0 concentration of RIM or MMI shows 20µM H₂O₂.

RIM degrades H₂O₂ in a dose response manner. Even MMI can degrade H₂O₂ although higher concentration of MMI is required. Accordingly, degradation of H₂O₂ by RIM can explain an inhibition of the LPO- H₂O₂ reaction.

### Human hypothyroidism caused by RIM

The data of RIM, *in vitro*, are very promising to be used as an antithyroid agent and the most important question is whether it inhibits thyroid hormone formation *in vivo.*

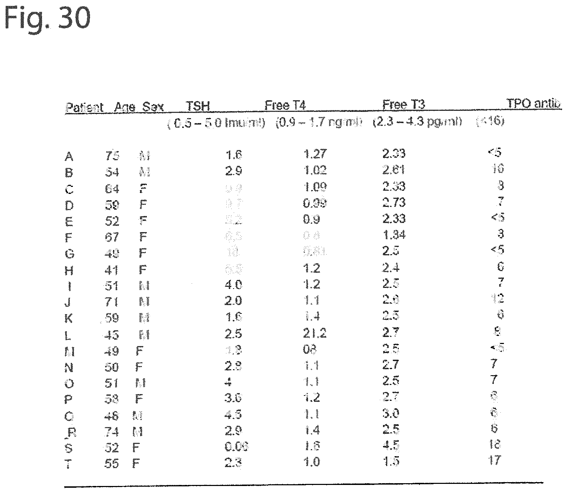

Shunichi Magara, MD in Japan, is using routinely RIM, 100mL of 3% RIM solution, in a treatment of recurrent cancer. A thyroid function is tested on randomly chosen patients who are taking this treatment, and results are shown in Fig. 30.

The therapy using RIM is based on the following reasons. Reactive oxygen species (ROS) increase in many types of cancer cells, and ROS increase a tumor angiogenesis. All patients who complete a chemotherapy or a radiation therapy for a long time are treated with RIM at least 2 months or longer. None of the patients had thyroid function test prior to the treatment because none of them had clinically suspected hypothyroidism. All patients were not suffering from a kind of sickness to cause non-thyroidal illness.

Six of 17 patients (35%) had hypothyroidism (4 sub-clinical hypothyroidism and 2 overt hypothyroidism) during treatment with RIM. The incidence of 35% hypothyroidism among randomly chosen 17 patients is unusually high comparing 6.5% prevalence of hypothyroidism in Japanese population. In addition, none of the six patients with hypothyroidism had Hashimoto's thyroiditis (the most common cause of hypothyroidism) based on the TPO antibody test. Other tests including CBC, liver and kidney function tests showed normal in all patients (not shown here). The results are promising for antithyroid action of RIM in humans.

However, if this is a potent antithyroid agent, all patients should develop a hypothyroidism. A relatively low incidence of a hypothyroidism (35%) suggest an important clue in a modification of administration method. Presumptively, an oral administration of RIM may not be so effective, but an amount of RIM to reach the thyroid gland may be an important issue. Topical application of RIM to the neck (thyroid gland area) or direct injection of RIM to the thyroid gland needs to be considered as one of effective RIM treatments. Interestingly, RIM is easily absorbed through skin

### Identification of the core component of RIM having reducing activity

Identification of the core component of RIM is crucial in this project. Since this experiment requires an expertise of inorganic chemistry and x-ray crystallography. Saeed I. Khan and JD. McCllough at UCLA have conducted collaboratively the works. The inventor of the present invention identifies the core elements focusing on sodium, potassium and sulfur in RIM. Specifically, a sulfur compound formed with sodium and potassium, or a double salt of NaKS, is a target.

### Analysis of crystalline RIM

Analysis of crystals from RIM was carried out by Saeed I. Khan, PhD at the JD. McCllough X-ray Crystallography Laboratory, UCLA. The first approach is to obtain a high quality single crystal using a variety of crystallization techniques. The idea is to selectively crystallize one of the many different phases of RIM. The inventor of the present invention applies a variety of different solvents (such as acetone, ethanol, methanol, formamide) to selectively extract the many different phases of the RIM and identifies these phases using both a single crystal method and a powder method.

A quality of crystal can be evaluated by looking at the diffraction spots, wherein an overlapped spot indicates multiple crystals appearing as if a single crystal. Only a good single crystal of the material is needed for a single crystal analysis. Bruker Smart 1000 Apex II (Bruker AXS Inc, Madison WI) is used for a single crystal analysis. For a powder X ray diffraction analysis, PANalytical X' Pert PRO diffractometer (Westborough, MA) is used.

### Analysis of amorphous RIM

RIM has amorphous components, non-crystalline material, and a measurement using ORP meter showed that water soluble amorphous materials retain a good reducing potential. Accordingly, there is a likelihood in which amorphous materials might be the core of the RIM molecule. Absence of a single spot of Na₂S and/or K₂S in the XRD RIM crystal analysis favors for an amorphous component as a major reducing source. Another likelihood is the formation of as yet to be identified a double salt of NaKS compounds, which may not form a crystal according to Dr. Kaesz's (collaborator) prediction.

An ion exchange or high pressure liquid chromatography is used to separate these sulfur containing compounds from water soluble RIM. The separated contents are analyzed as to reducing potential by the ORP meter. Target samples retaining a great reducing potential are analyzed by crystallization followed by an x-ray diffraction analysis or an element analysis.

On analysis, it should be notable that sulfur compounds based on mineral contents of RIM and reducing potentials of sulfur compounds. During the process of RIM formation, heating at 2000°C easily melts sulfur allowing Na and K to interact with liquid sulfur. Entrapped Na and K in liquid sulfur at high temperature may produce unexpected sulfur compounds, since RIM, despite highly potent reducing activity of sulfur compounds, does not cause side-effects.

### Experiments using human thyroid follicles or thyroid cell lines

### (1) Examination of the effects of RIM on H₂O₂ generation at a physiological unit of thyroid follicles

Procedures: Frozen human thyroid follicles from patients with Graves' disease are obtained from Dr. Andrew Gianoukakis, Harbor-UCLA hospital. Culturing follicles requires 1% agar coating in the bottom of a culture dish. Without this coating, follicles are broken into monolayer cells.

Human follicles are maintained in 2mL of 6H medium (TSH) or 5H medium (no TSH) in 6-well plates. For measurement of H₂O₂, follicles are preincubated in the presence of 1.5mM CaCl₂ and 8mM glucose for 1 day prior to the assay. Then, the medium is changed to 1.2mL HBSS containing 1nM PMA (direct stimulator of DUOX2), 1.5mM CaCl2 and 8mM glucose, TSH (0 to 1 mU/mL) with or without RIM (0 to 20µL of 1 % RIM), pH 7.4.

After this mixture is incubated for 1 hour in a CO₂ incubator, 50µL of medium are taken into 96-well plate for measurement H₂O₂ levels by Amplex reagent mixture using the microplate reader at the absorbance of 560nm. The amount of H₂O₂ generated will be expressed micro-mole H₂O₂/mg protein.

### (2) Examination of RIM effects on iodide organification in thyroid cells

One of the advantages of thyroid follicles is to demonstrate iodide organification. Thyroid cells cultured in monolayer are unsuitable for iodide organification because of lack of three dimensional follicular shapes. The purpose of this study is to examine whether RIM inhibits iodide organification at cellular level or not.

Human thyroid follicles in 12-well dish plate are cultured in 5H medium and 6H medium as described above. On the day of experiment, the medium is changed to HBSS medium, pH 7.4, containing 1.5mM CaCl₂, 8mM glucose, 1nM PMA, 0 to 1mU/mL TSH, and 0 to 20µL RIM. Then, the mixture of 1251-iodide (30,000cpm) and10nM KI, iodide carrier, are added to the incubation medium. Incubation is carried out for 2 hrs at 37°C in a CO₂ incubator. The entire follicles in the culture medium are sonicated and centrifuged at 5000 x g for 10 min.

### (3) To determine whether RIM primarily affects DUOX- or NOX4-mediated H₂O₂ generation by knockdown of NOX4.

First, NOX4 and DUOX2 knockdown experiment is carried out. It is unknown how NOX4 and DUOX2 contribute to H₂O₂ generation in thyroid cells. In addition, it is measured whether RIM primarily affects DUOX- or NOX4-mediated H₂O₂ generation. An approach is to knock down NOX4 or DUOX2 gene and to examine H₂O₂ generation with or without RIM. Pooled four NOX4 siRNA or DUOX2 siRNA (Dharmacon,Chicago. IL, siGENOME, SMART pool) and scrambled siRNA control (Dharmacon) are used. And monolayer human thyroid cells derived from human follicles are used. Transfection of primary culture of human thyroid cells are carried out using the JetPEI transfection medium (Ployplus, New York, NY) as Weyemi et al. Human thyroid cells are cultured without TSH for 3 days, followed by incubation of cells with the pooled NOX4 or DUOX2 siRNA- JetPEI complex for 8 hrs. The knock down efficiency will be checked by real-time QPCR and immunohistochemical stain of NOX4 or DUOX2 in thyroid cells. H₂O₂ generation is carried out by the Amplex Redmethod (Invitrogen) using following samples.

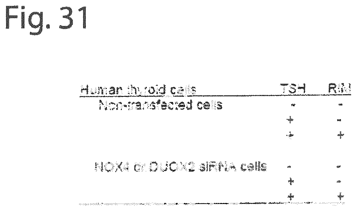

Localization of cellular H₂O₂ and O₂ in cultured cell and the amount of their production are examined using the specific H₂O₂ HyPer protein (Wako Chemical Inc, Richmond, VA) and O₂ - specific mitoSOX Red probe (Invitrogen). The results are shown in Fig. 31.

### (4) Western blot examination of DUOX and NOX4 molecule after exposure to RIM

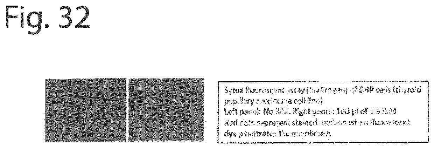

This is to examine whether RIM directly affects membrane DUOX and intracellular NOX4 molecules. Since RIM affects the plasma membrane (referring to Fig. 32), RIM appears to cross the plasma membrane as evidenced by increasing intracellular reducing potential after exposure to RIM in BHP cells (unpublished data). Human follicles are incubated in 6H medium with or without 1 % RIM for 2 hrs in 6-well culture dishes. Plasma membrane fraction form cultured follicles are obtained from follicles pooled by Song et al. Membrane fraction are stored at -80°C until use for the Western blotting. Rabbit polyclonal antibodies for DUOX2 and NOX4 are obtained from Dr. Jacqueline Van Sande. For internal control of membrane protein, caveolin-1 rabbit polyclonal antibody (Cell SignalingTechnology, Danvers, MA) is used.

### Effects of RIM on cAMP generation

This experiment is to examine whether RIM affects the most important signal of thyroid hormone formation from the membrane-TSH-mediated cAMP generation. The generation of cAMP is initiated when a ligand (TSH) binds to a G protein-coupled receptor, stimulating the enzyme adenylyl cyclase (AC) to catalyze the cyclization of ATP. If this signal is affected by RIM, this could be another mechanism of inhibition of thyroid hormone formation.

As a procedure, FRTL-5 cells are used.

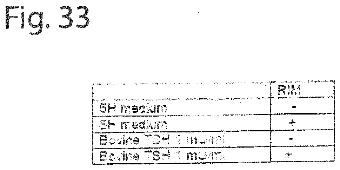

Cells in a 24-well plate are cultured with 5H medium (without TSH) for 5 days in a CO₂ incubator. On the day of experiment, the culture medium is changed to HBSS medium, pH 7.4, and 50µL 1% RIM will be added to some groups. After cells are exposed to RIM for 10 min, bovine TSH (1 mU/m) or 200µM forskolin (adenylyl cyclase stimulator) is added to some of the groups. After incubation for 1 hr, cells are lysed with 0.1 M HCl and intracellular cAMP levels are measured using Parameter cAMP assay kit (R&D Inc. Minneapolis MN) according to the manufacturer's instruction. The results are shown in Fig. 33.

### Cell free experiments

Comparison of antithyroid potency of RIM with MMI is carried out. To establish the relative potency of RIM as an antithyroid agent, it is compared with the antithyroid potency of MMI by the following two methods.

Radioiodination of BSA is carried out in the presence of LPO, H₂O₂, 1251-iodide and BSA. To this reaction mixture, RIM or MMI having different concentrations are added, and the percentage of radioiodination of BSA by RIM or MMI are determined. The antithyroid potency is determined by calculating concentrations at which 50% inhibition of iodination by RIM or MMI occurs. Effects of RIM or MMI on compound II levels, oxidizing product of peroxidase- H₂O₂ reaction for thyroid hormone formation, are examined by the spectrum analysis of compound II (at 430nm).

The first experiment is premixing RIM or MMI before addition of LPO and H₂O₂. The reaction mixture contains RIM or MMI and LPO. An absorbance at 430nm are recorded immediately after addition of H₂O₂ by Shimadzu spectrometer.

The second experiment involves preformation of compound II by adding LPO and H₂O₂ first, followed by addition of RIM or MMI. Compound II levels are recorded immediately after mixing LPO and H₂O₂. Then, RIM or MMI are added and changes in compound II level are recorded for 3 min using a kinetic mode of spectrophotometer.

### Animal experiments

The main objectives in animal experiments are to demonstrate whether RIM induces reversible or irreversible hypothyroidism, goiter formation or thyroid atrophy. In addition, transdermal effects of RIM are examined considering the possibility of a topical application of RIM for treatment of Graves' disease. Sprague Dawly rats are used in the experiment because they are one of the commonly used animals for thyroid experiments. The gender of rats is not critically important. Rats with age between 1-month and 2-month old are used because older rats may have spontaneous goiter or tumor in the thyroid gland. Rats are housed in the designated animal facility of GLA VA Hospital (building 113), where animals are taken care by qualified animal staff members and veterinarians. Animal experiments are carried out according to the guidelines established by GLA-VA Institutional Animal Care and Use Committee (IACUC).

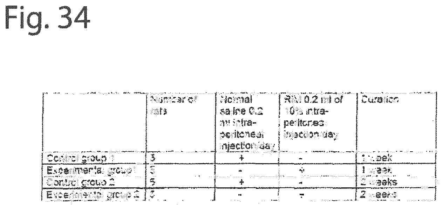

Pilot experiments are carried out to obtain the following information: an optimal dose of RIM, an optimal duration of treatment and the number of rats to be used by statistical power analysis. Further, StatMate (GraphPad Software Inc, La Jolla, CA) is used to analyze statistical power. The results are shown in Fig. 34.

At the end of experiments, rats are sacrificed in a CO₂ chamber. Blood samples are obtained immediately by cardiac puncture for measurement of serum TSH, free T4, CBC, kidney and hepatic function at Antech Diagnostics, Irvine, CA. The neck is dissected to expose the thyroid gland for taking pictures of thyroid size, and the thyroid gland is removed. The thyroid gland is weighed, sliced and stained with hematoxyline-eosin for microscopic examination. Portions of rat thyroid tissue are used to analyze T3 and T4 content in the thyroid gland. For measurement of thyroid hormone content, thyroid tissues are sonicated in PBS containing protease inhibitor cocktails (Sigma-Aldrich), followed by pronase digestion (Calbiochem, La Jolla, CA) as the inventor of the present invention described before. Then, thyroid hormone is extracted with ethanol.

Total T3 and T4 contents in thyroid tissue are measured by a radioimmunoassay. Also, an aliquot of sonicated thyroid is used for measurement of tissue reducing potential by the ORP meter. A comparison of thyroid reducing potentials between control and RIM treated groups should disclose the presence of RIM effects on thyroid tissues.

Pilot experiments should provide the inventor of the present invention with the following information; an optimal dose of RIM to be administered in the future, an optimal duration of treatment, and a statistical out-put power to determine a number of rats to be used for future experiments. An Oral administration of RIM in drinking water is not considered because an oral intake of RIM is variable due to an unpredictable absorption of RIM from a gastrointestinal tract. A measurement of total T3 and T4 can be difficult from small rat thyroid tissues. Pooling thyroid tissues are tried to circumvent this problem, Dr. GH Pez, staff and pathologist as collaborators examine rat thyroid histology. The practical problem is a frequent injection of RIM. An alternative choice is a pellet implantation under the skin, wherein a custom pellet provided by Innovative Research of America is used to releases drugs slowly. Also, an additional advantage of using pellet is applied in the neck as a preferred implant site, by which RIM can reach preferably and effectively to the thyroid gland. This method is worth to try as a pilot experiment although no preliminary study has been done.

### Examination of reversible or irreversible effects of RIM on the thyroid gland

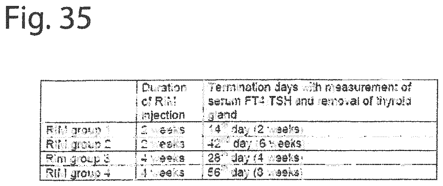

When it is used in human, it is a critical question whether RIM causes permanent or reversible hypothyroidism or not. Experiments on rats are carried out to answer this question. Further, an experiment plan is shown in Fig. 35.

The dose of RIM and the number of animals are determined after the pilot experiment. No control group (NS injection) is included in this experiment.

### Transdermal effect of RIM

This is to examine whether RIM penetrates skin and reaches to subcutaneous tissues on a topical application, and the topical application of RIM to the neck, where Graves' thyroid gland is easily palpable, is considered. This agent is easily absorbed through the skin. As a Procedures, firstly, Sprague-Dawley rats (n=5) are used in this experiment. Application areas for RIM and normal saline are shaved by an electric shaver to expose skin. RIM (5% solution) or normal saline (control) are applied to the designated areas twice a day using a small painting brush. The experiment is carried out for 1 week.

After the rats are sacrificed, subcutaneous tissue are separated from skin and weighed, and then sonicated in PBS. An sonicated aliquot is used for measurement of reducing potential by the ORP meter. ORP readings on subcutaneous tissues from control and RIM sites are compared. The depth of RIM penetration is examined by measurement of reducing potential on subcutaneous tissues of different portions (superficial vs. deep tissues).

A topical application of RIM to the neck of rat sounds the most practical approach to examine the effects of topical RIM on serum level of TSH/free T4. However, a rat thyroid gland is covered by muscles in contrast to a human thyroid gland that is easily palpable. Also, a shaving of neck area is not easy on a rat unless a light anesthesia is introduced.

Next, the inventor of the present invention describes other pharmacological effect and cosmetic effects of a biological inorganic compound complex having reduced oxygen and high reducing ability and consisting of positive atomic valence elements such as sodium and potassium and sulfur.

### Other pharmacological effects

A biological inorganic compound complex having reduced oxygen and high reducing ability and consisting of sodium, potassium and sulfur can be used to relieve an oxidation stress, improve and recover disorders by utilizing potent reducing ability thereof. For example, in case of a skin inflammation such as atopic dermatitis and athlete's foot, RIM aqueous solution can be topically applied so that the inflammation of skin inflammation can be relieved by potent reducing ability. Further, it has an effect on burns and hemorrhoids and so on, and damaged parts can be recovered by potent reducing ability. Further, it has an effect on pneumonia, swelling, pain and itchiness, and a symptom can be suppressed by an application on body surface.

Since a mineral balance of RIM and body fluid is mostly common, RIM can be applied as drops by adjusting pH of 0.6% - 0.8% solution of RIM.

### Cosmetic effects

A biological inorganic compound complex having reduced oxygen and high reducing ability and consisting of positive atomic valence elements such as sodium and potassium and sulfur can be applied to mix into a soap, a cream and a lotion and a serum to provide a comfortable stimulation and a pleasurable sensation on use, maintain skin moisture, and provide a moisture. Further, if applied on oxidized skin, such a skin condition can be recovered and skin damage due to an ultraviolet ray can be prevented by potent reducing ability. In addition, it has a hair growth effect.

### Application in agriculture

RIM and BIE having potent reducing ability can be applied to plant and crop cultivations.

Seeds to be put are immersed in 0.01% - 0.1% RIM solution for one day so that chemicals attached to the seeds can be eliminated and reducing ability and a mineral balance of RIM can be immersed in the seeds. If it is used as a fertilizer, it can provide enough minerals to plants and crops. Further, its aqueous solution is sprayed onto bodies of plants and crops, it may help to prevent plant diseases and control pests Further, if it is sprayed onto harvested crops and flowering grasses, they are washed and remain in fresh. In addition, even if human intakes as is, it is advantageous that it does not affect a human.

### Animal husbandry and aqua-farming

RIM and BIE having potent reducing ability can be applied to animal husbandry for cow, pig and chicken, and aqua-farming for seafood. If the novel substance is mixed with animal feed, farming safely and disease-resistive animals and fishes and shellfishes can be farmed without a synthetic feed and antibiotics.

### Application to foods

RIM having potent reducing ability can be applied to foods. For example, RIM can be used to maintain food's freshness, extract umami, and wash foods. In addition, if a RIM aqueous solution is sprayed on foods or foods and immersed, potent reducing ability thereof can sterilize foods so that a food poisoning can be prevented.

Next, the inventor of the present invention describes an apparatus to produce mineral water by utilizing BIE having potent reducing ability.

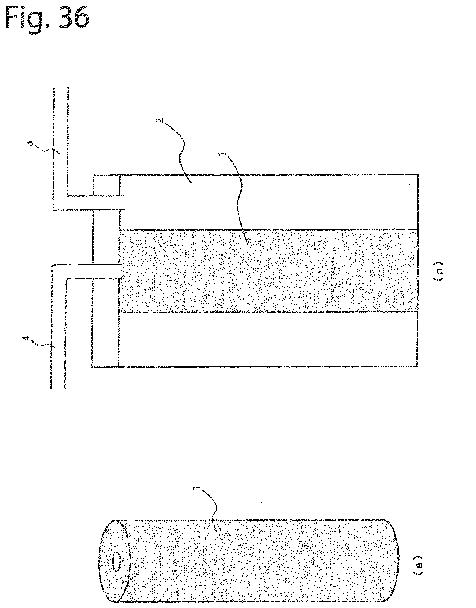

Fig. 36(a) illustrates a structure of a mineral elution filter, and 36(b) is a cross section view illustrating a main body of an apparatus to produce mineral water.

At the beginning, first burn wild grasses, herbaceous plants, tree leaves and seaweeds to produce ashes and melt it at 2000°C, and then make it powdery, dissolve it in water and then filter remove water soluble minerals to obtain water-insoluble plant minerals. Pulverize the water-insoluble plant minerals to produce fine powder having a smaller size, not larger than 10µm, which is used as a raw material of mineral elution filter 1.

A mineral elution filter 1 is made of nylon with either fibrous form or net-like structure on which powdery materials produced after melting at 2000°C as described above and insoluble pant minerals are adsorbed by such as heating. Further, such as activated carbon, silver having a sterilization effect and ores can be added and they can be adsorbed. The mineral elution filter 1 is formed as a hollow cylinder by layering plural filters and set in a housing 2 having a larger diameter than a diameter of filter 1.

A supply pipe 3 to provide water and an intake pipe 4 to take out produced mineral water are connected to housing 2. First, tap-water is supplied from supply pipe 3 into housing 2 (outside of mineral elution filter 1). Supplied water passes through inside mineral elution filter 1 and mineral water is obtained from intake pipe 4 in a hollow part.

In addition, mechanisms to supply tap-water to housing 2 and to take out mineral water from it can be heretofore known technology.

Chlorine contained in tap-water can be eliminated by adsorbing on mineral elution filter 1 when tap-water supplied to housing 2 passes through mineral elution filter 1. Further, minerals pre-adsorbed on mineral elution filter 1 elute and allow producing water rich in minerals. Further, oxidative tap-water is reduced by above RIM having potent reducing ability and mineral water having alkali property can be produced by lowering oxidation-reduction potential.

Needless to say, the produced water is the most suitable water for living organisms and can be used as service water for a cultivation of plants and crops and animal husbandry, and in addition, it is suitable in foods and to wash foods because of safeness in human.

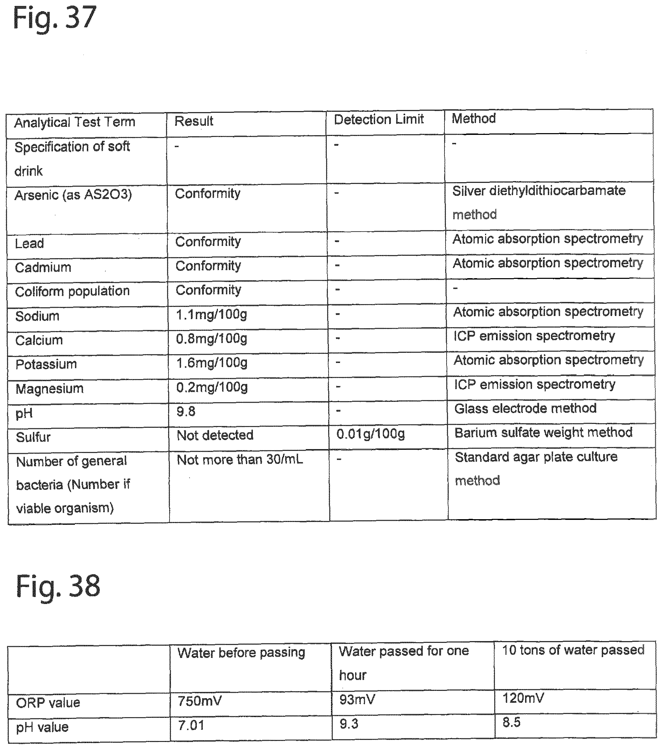

As shown in Fig. 37, mineral contents are measured in produced mineral water contains; potassium 1.6mg/100g, sodium 1.1mg/100g, calcium 0.6mg/100g, and magnesium 0.2mg/100g; pH is 9 thereof, and indeed the mineral water and body fluid have the common mineral balance.

### Example of experiment

### Performance testing of 50cm of mineral elution filter (with activated carbon)

Water tested: Tokyo Metropolitan tap-water
Water volume: water flow rate 8L/min on passing through a filter

The results are shown in Fig. 38.

According to the above example, tap-water passed through a mineral elution filter clearly shows lower ORP values and having reducing ability, and pH thereof turned to alkali.

Further, if polluted air is passed through mineral elution filter 1 installed with air-purifier, air conditioner and ventilation, the air can be purified. When polluted air passes through the filer 1, pollutants are adsorbed on the fibrous form or net-like structure filter 1 and at the same time pollutants are reduced by mineral having reducing property and air can be cleaned. Such apparatus is installed in an animal rearing room, a plant production room, a smoking room, a toilet and so on to provide such effects.

### Reference of sign

1. Mineral elution filter
2. Housing
3. Supply pipe
4. Intake pipe

## Claims

1. A water-soluble inorganic compound complex for use in treating hyperthyroidism, wherein an aqueous solution of said water-soluble inorganic compound complex has an oxidation-reduction potential (ORP value) of between - 600mV and -700 mV, whereas a powder obtained by completely drying the aqueous solution of said water-soluble inorganic compound complex has an oxidation-reduction potential of between -80 mV and -300mV, said inorganic compound complex comprising sodium and potassium having positive atomic valence, chlorine having negative atomic valence and S, CO₃²⁻, and SO₄²⁻, wherein a powder of said water-soluble inorganic compound complex contains X-ray powder diffraction peaks assigned to K₃Na(SO₄)₂, Na₄(SO₄)_{1.39}(CO₃)_{0.61}, Na₄(SO₄)_{1.45}(CO₃)_{0.55}, Na₄(SO₄)_{1.51}(CO₃)_{0.49}, KNa(SO₄), KHS, S, Na₂S, KNaS, SiO₂, NaS₂, S₆, Al(OH)₃ and Na₂S₄.

## Patentansprüche

1. Wasserlöslicher anorganischer Verbindungskomplex zur Verwendung beim Behandeln von Hyperthyreoidismus, wobei eine wässrige Lösung des wasserlöslichen anorganischen Verbindungskomplexes ein Oxidations/Reduktionspotential (ORP-Wert) von zwischen -600 mV und -700 mV aufweist, wohingegen ein Pulver, das durch vollständiges Trocknen der wässrigen Lösung des wasserlöslichen anorganischen Verbindungskomplexes erhalten wird, ein Oxidations/Reduktionspotential von zwischen -80 mV und -300 mV aufweist, wobei der anorganische Verbindungskomplex Natrium und Kalium mit einer positiven Atomwertigkeit, Chlor mit einer negativen Atomwertigkeit und S, CO₃²⁻ und SO₄²⁻umfasst, wobei ein Pulver des wasserlöslichen anorganischen Verbindungskomplexes Pulver-Röntgenbeugungspeaks enthält, die K₃Na(SO₄)₂, Na₄(SO₄)_{1,39}(CO₃)_{0,61}, Na₄(SO₄)_{1,45}(CO₃)_{0,55}, Na₄(SO₄)_{1,51}(CO₃)_{0,49}, KNa(SO₄), KHS, S, Na₂S, KNaS, SiO₂, NaS₂, S₆, Al(OH)₃ und Na₂S₄ zugeordnet werden.

## Revendications

1. Complexe de composé inorganique soluble dans l'eau pour une utilisation dans le traitement de l'hyperthyroïdie, dans lequel une solution aqueuse dudit complexe de composé inorganique soluble dans l'eau possède un potentiel d'oxydation-réduction (valeur ORP) comprise entre -600 mV et -700 mV, alors qu'une poudre obtenue par le séchage complet de la solution aqueuse dudit complexe composée inorganique soluble dans l'eau possède un potentiel d'oxydation-réduction compris entre -80 mV et -300 mV, ledit complexe de composé inorganique comprenant du sodium et du potassium possédant une valence atomique positive, du chlore possédant une valence atomique négative et S, CO₃²⁻ et SO₄²⁻, dans lequel une poudre dudit complexe de composé inorganique soluble dans l'eau contient des pics de diffraction de rayons X sur poudre attribués à K₃Na(SO₄)₂, Na₄(SO₄)_{1,39}(CO₃)_{0,61}, Na₄(SO₄)_{1,45}(CO₃)_{0,55}, Na₄(SO₄)_{1,51}(CO₃)_{0,49}, KNa(SO₄), KHS, S, Na₂S, KNaS, SiO₂, NaS₂, S₆, Al(OH)₃ et Na₂S₄.
